# FASCICULE DE BREVET EUROPEEN

(11) **EP 3 062 632 B1**
(45) Date de publication et mention de la délivrance du brevet: **06.03.2019**
(21) Numéro de dépôt: 14796226.0
(22) Date de dépôt: 13.10.2014
(51) Int. Cl.: A23J 3/08, A61F 13/15, C08B 37/00, C08H 1/00, C12P 19/04

(54) **POLYMERE PHÉNOLIQUE A LIAISONS BIARYLES 5-5, PROCÉDÉ POUR SA PRÉPARATION ET UTILISATIONS**
PHENOLPOLYMER MIT 5,5'-BIARYLBINDUNGEN, VERFAHREN ZUR HERSTELLUNG DAVON UND VERWENDUNGEN DAVON
PHENOL POLYMER WITH 5,5'-BIARYL BONDS, METHOD FOR PREPARING SAME, AND USES THEREOF

(30) Priorité: 14.10.2013 FR 1359948
(43) Date de publication de la demande: 07.09.2016
(73) Titulaire: Institut National de la Recherche Agronomique, 75007 Paris Cedex 07 (FR); INST Sciences IND Vivant Environnement, 75005 Paris 5 (FR)
(72) Inventeur: ALLAIS, Florent, F-94240 L' Hay-les-Roses (FR); PION, Florian, F-22190 Plerin (FR); REANO, Armando, 78500 Sartrouville (FR); DUCROT, Paul-Henri, F-91430 Igny (FR); SPINNLER, Henry Eric, F-92310 Sèvres (FR)
(74) Mandataire: Ipside
(86) Numéro de dépôt international: PCT/FR2014/052604
(87) Numéro de publication internationale: WO 2015/055936

(56) Documents cités:
- EP-A1- 1 169 922
- EP-A1- 1 731 043
- WO-A1-96/03440
- WO-A1-97/27221
- WO-A1-2004/083256
- WO-A2-03/037829

## Description

La présente invention s'inscrit dans le domaine des polymères phénoliques. Plus particulièrement, elle concerne un polymère phénolique, dont les monomères de base sont des macropolyphénols, en particulier des macrobisphénols, et dans lequel les liaisons entre monomères sont exclusivement de type biaryle 5-5. L'invention concerne également un procédé pour la synthèse d'un tel polymère et une composition le contenant, ainsi que l'utilisation d'un tel polymère phénolique, notamment en tant qu'agent antioxydant, agent antiradicalaire, agent antimicrobien, agent chélatant ou agent plastifiant.

L'emploi des biotechnologies blanches, c'est-à-dire mettant en oeuvre un système biologique, en particulier un système enzymatique, pour la synthèse de molécules, présente un intérêt croissant, par rapport aux procédés chimiques classiques, du fait du caractère économique et respectueux de l'environnement de ces systèmes. Cet intérêt est d'autant plus important lorsqu'il est possible d'utiliser, en tant que matières premières pour la synthèse des molécules, des synthons biosourcés, par exemple issus de la biomasse végétale.

De nombreuses études ont ainsi porté, durant ces dernières années, sur la mise en oeuvre d'enzymes pour la catalyse de réactions qui étaient conventionnellement réalisées par des procédés chimiques. Parmi ces études, certaines se sont intéressées aux enzymes du type oxydase, et plus particulièrement aux laccases, pour la réalisation de couplages oxydatifs de composés à groupement phénolique, polyphénolique ou aniline.

Le document WO 96/03440 décrit un procédé de réticulation, catalysée par une laccase, de polysaccharides phénoliques comportant un dérivé de l'acide férulique en tant qu'unité constitutive. Le document WO 97/27221 divulgue également un procédé de réticulation, catalysée par une oxydase et une hydrolase d'ester carboxylique, de polysaccharides à substituants basés sur l'acide férulique.

Le document WO 03/037829 divulgue un procédé de synthèse d'oligophénols sur un support solide, par oligomérisation catalysée par une oxydase.

Le document EP 1 731 043 divulgue des conjugués de polymères comportant des substituants phénoliques tels que l'acide férulique, l'acide caféique, l'acide coumarique, l'acide vanillique et l'acide cinnamique, et leur procédé de préparation par réticulation par catalyse enzymatique.

Le document EP 1 169 922 divulgue un procédé de synthèse de macromolécules mixtes par réticulation, par une enzyme et un agent oxydant, d'une protéine ou d'un peptide et d'un polymère comportant des substituants dérivés d'acides carboxyliques contenant des groupes phénoliques.

Le document WO 2004/083256 divulgue un polymère, obtenu par réaction catalysée par une enzyme oxydative, comportant au moins deux blocs choisis parmi les protéines, les glycérides et les polysaccharides, liés de façon covalente par des résidus phénoliques, ces derniers comportant un seul noyau phénolique.

C'est notamment également le cas de l'étude décrite dans la publication de Kunamneni et al., 2008 (Microbial Cell Factories, 7:32).

Cependant, comme en témoigne notamment cette publication, dans laquelle il est montré que l'oxydation de l'oestradiol par une laccase mène à la formation de quatre composés dimères différents, le couplage phénolique oxydatif catalysé par les enzymes de type oxydase présente une régiosélectivité extrêmement difficile à contrôler, le couplage des motifs phénoliques l'un à l'autre pouvant s'effectuer sur plusieurs sites respectifs des molécules de base.

Or, il serait intéressant de disposer de composés polymères phénoliques obtenus par couplage de molécules phénoliques, notamment de macropolyphénols, et en particulier de macrobisphénols, ce couplage étant réalisé de préférence de manière enzymatique, et dans tous les cas de manière simple et contrôlée de sorte à assurer la formation d'un unique produit polymère et la présence, dans ce polymère, de nombreuses fonctions phénoliques libres. En effet, cette présence, associée notamment au caractère aromatique du polymère et à son poids moléculaire élevé, feraient de ce dernier un candidat idéal en tant qu'agent antioxydant, antiradicalaire, antimicrobien, chélatant et/ou plastifiant. C'est cet objectif que vise la présente invention.

De manière tout à fait avantageuse et surprenante, il a maintenant été découvert par les présents inventeurs que cet objectif pouvait être atteint, et que des composés polymères présentant de nombreuses fonctions phénoliques libres, et dotés de propriétés antioxydantes, antiradicalaires, antimicrobiennes, chélatantes et/ou plastifiantes importantes, pouvaient être obtenus de manière simple et contrôlée par oligomérisation catalysée par une enzyme de type oxydase, à partir de macropolyphénols répondant à une structure particulière.

Ainsi, il est proposé par les présents inventeurs un polymère phénolique susceptible d'être obtenu par oligomérisation catalysée par une enzyme de type oxydase, en particulier une laccase, d'un ou de plusieurs macropolyphénol(s) répondant chacun à la formule générale (I) : 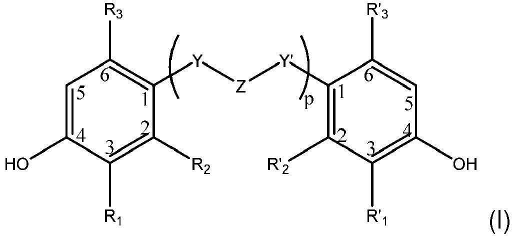 dans laquelle :
p représente un nombre entier compris entre 1 et 30,
R₁, R'₁, R₂, R'₂, R₃ et R'₃, identiques ou différents, représentent chacun un atome d'hydrogène, un atome de chlore, un atome de brome, un atome d'iode, un atome de fluor, un groupe alkyle, benzyle, Xalkyle, le cas échéant substitué, Xbenzyle, le cas échéant substitué, Xacyle, B(OR')₂, NHR', NO₂, SR'O ou SO₂R',
   où X représente N, O, S ou P
   et R' représente un groupe alkyle ou un groupe aryle,
R₁ et R'₁ ne représentant pas un atome d'hydrogène,
Y et Y', identiques ou différents, représentent chacun :
   soit, un atome d'oxygène, un atome de soufre ou un groupe déconjuguant ne comportant ni noyau époxyde, ni noyau aziridine, ni groupement phénol qui ne soit pas substitué sur tous ses atomes de carbone,
   soit, un groupe répondant à la formule (II) : 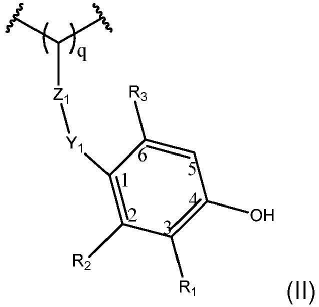
   dans laquelle :
   q représente un nombre entier compris entre 1 et 8,
   Y₁ représente un atome d'oxygène, un atome de soufre ou un groupe déconjuguant ne comportant ni noyau époxyde, ni noyau aziridine, ni groupement phénol qui ne soit pas substitué sur tous ses atomes de carbone,
   Z₁ représente un hétéroatome ou un groupe espaceur ne comportant ni noyau époxyde, ni noyau aziridine, ni groupement phénol qui ne soit pas substitué sur tous ses atomes de carbone, ni groupe alkényle, ni groupe alkynyle,
   et R₁, R₂ et R₃ sont tels que définis ci-avant,
   et Z représente :
   soit, un hétéroatome ou un groupe espaceur ne comportant ni noyau époxyde, ni noyau aziridine, ni groupement phénol qui ne soit pas substitué sur tous ses atomes de carbone, ni groupe alkényle, ni groupe alkynyle,
   soit, un groupe répondant à la formule (III) :

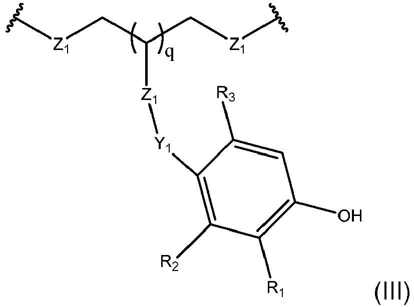

   dans laquelle q est un nombre entier compris entre 1 et 8, et R₁,
   R₂, R₃, Y₁ et Z₁ sont tels que définis ci-avant,
   les liaisons entre les fragments macropolyphénol de formule générale (I) au sein dudit polymère étant exclusivement des liaisons biaryle 5-5.

Dans la suite de la présente description, on désignera le macropolyphénol de formule (I) par le terme macrobisphénol lorsque Y et Y' ne représentent pas un groupe répondant à la formule (II) ci-dessus et Z ne représente pas un groupe répondant à la formule (III) ci-dessus.

On entend dans la présente description, par groupe déconjuguant, un groupe ne comportant pas de liaison conjuguée avec le noyau phénolique, et réalisant ainsi une rupture de conjugaison entre le noyau phénolique et le groupe Z, par rupture du recouvrement des orbitales pi entre ces derniers.

Le terme polymère est ici entendu pour englober tant les polymères *stricto sensu,* c'est-à-dire formés à partir d'un seul et même monomère, que les copolymères, en particulier les copolymères statistiques, formés à partir d'une pluralité de monomères, répondant cependant chacun à la formule générale (I), par exemple de 2 ou 3 monomères différents, voire plus.

Le polymère selon l'invention peut aussi bien être de type linéaire que ramifié, et notamment réticulé.

Par l'expression « ne comportant (...) ni groupement phénol qui ne soit pas substitué sur tous ses atomes de carbone », on entend que Z, Z₁, Y, Y', Y₁ peuvent comporter un groupement phénol, mais uniquement si tous les atomes de carbone du cycle aromatique y sont substitués, c'est-à-dire sont liés à un atome autre qu'un atome d'hydrogène.

De manière tout à fait avantageuse et surprenante, alors qu'on aurait pu penser, comme suggéré notamment par la publication de Kunamneni et al., 2008, que la polymérisation entre les macropolyphénols de formule générale (I) s'effectuerait entre plusieurs sites différents des molécules, et donnerait lieu à différents types de couplage, en particulier, outre des couplages biaryle de type 5-5, des couplages biaryle de type 1-1, 5-1 ou encore 4-*O*-5, le composé polymère selon l'invention ne comporte qu'un seul type de liaison entre les fragments macropolyphénols de formule générale (I) qui en forment les monomères constitutifs, plus précisément une liaison biaryle 5-5. Cette liaison n'affecte avantageusement pas les fonctions phénoliques des macropolyphénols, qui restent libres et donc réactives.

En outre, alors qu'on aurait également pu penser que la réaction de couplage enzymatique se serait effectuée entre deux noyaux phénoliques du même fragment macropolyphénol de formule générale (I), une telle réaction intramoléculaire étant favorisée cinétiquement par une grande proximité spatiale des radicaux, il a été constaté par les présents inventeurs qu'au contraire, et de manière inattendue, il se produisait sensiblement exclusivement dans le milieu réactionnel un couplage intermoléculaire, conduisant à la formation d'un polymère.

Dans les modes de réalisation dans lesquels le ou les monomères de départ sont des macrobisphénols, le polymère selon l'invention présente en outre tout à fait avantageusement une structure homogène, linéaire ou cyclique, de degré de polymérisation contrôlée, et de nombreuses fonctions phénoliques libres distribuées uniformément le long de la chaine polymère. Ce polymère présente en particulier de ce fait de fortes propriétés antioxydantes, plastifiantes, antiradicalaires et antimicrobiennes. Sous forme cyclique, ce polymère présente également des propriétés chélatantes.

Lorsque Z représente un groupe répondant à la formule (III) ci-avant, ou Y ou Y' représente un groupe répondant à la formule (III) ci-avant, c'est-à-dire qu'au moins un macropolyphénol de formule générale (I) n'est pas un macrobisphénol, le polymère selon l'invention présente une structure ramifiée. Un tel polymère présente également avantageusement un nombre important de fonctions phénoliques libres, et, en particulier, de bonnes propriétés plastifiantes, notamment du fait de son caractère aromatique et de son poids moléculaire élevé.

Le polymère selon l'invention constitue notamment un substitut avantageux au bisphénol A et ses dérivés.

Préférentiellement, chaque macropolyphénol de formule générale (I) est un composé biosourcé, notamment issu de la biomasse végétale, et en particulier de la biomasse lignocellulosique. Le polymère selon l'invention, formé à partir d'un tel monomère de base, présente alors avantageusement une nature renouvelable et une toxicité potentiellement réduite ou nulle, qui le rend tout à fait approprié pour une mise en oeuvre dans des domaines tels que celui de l'alimentaire ou de la cosmétique.

Des exemples de tels macropolyphénols biosourcés sont notamment décrits dans la publication de Pion et al., 2013 (RSC Advances, 3, 8988-8997).

Dans la formule générale (I) ci-dessus, R₁ et/ou R'₁ représente(nt) de préférence un radical hydrocarboné saturé, linéaire ou ramifié, comportant de 1 à 5 atomes de carbone, par exemple un radical *tert*-butyle, ou un groupe OR₄, où R₄ représente un radical hydrocarboné saturé, linéaire ou ramifié, comportant de 1 à 5 atomes de carbone, par exemple un radical méthyle.

Préférentiellement, R₂, R₃, R'₂ et/ou R'₃ représente(nt) un atome d'hydrogène.

Dans la formule générale (I) ci-dessus, Y et Y' peuvent représenter tout groupe déconjuguant dépourvu de noyau époxyde, de noyau aziridine et de noyau phénol qui ne soit pas substitué sur tous ses atomes de carbone. Il est du ressort de l'homme du métier d'identifier les groupes répondant à une telle définition. Par exemple, Y et/ou Y' peut/peuvent représenter un groupe de formule générale (V) :

(CH₂)ₘ-X'- (V)

dans laquelle m est compris entre 1 et 5
et X' représente un atome d'oxygène ou un atome de soufre ou un groupe choisi parmi les groupes : 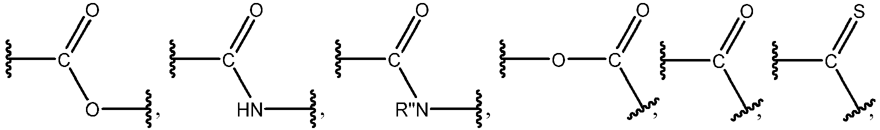 NR", NH ou SO₂, où R" représente un groupe alkyle ou un groupe aryle.

Y₁ répond également de préférence à une telle définition.

Dans des variantes de l'invention, dans la formule générale (I), Y et Y', et le cas échéant Y₁, représentent chacun un groupe de formule générale (VI) :

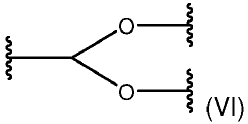

Le polymère phénolique selon l'invention est alors avantageusement biodégradable, notamment en milieu aqueux.

Z représente quant à lui de préférence un groupe hydrocarboné saturé, linéaire ou ramifié, le cas échéant substitué, comportant de 1 à 6 atomes de carbone, pouvant comporter un ou plusieurs hétéroatomes, ou un groupe hydrocarboné cyclique saturé, le cas échéant substitué, comportant de 1 à 6 atomes de carbone, pouvant comporter un seul cycle ou plusieurs cycles condensés, et pouvant comporter un ou plusieurs hétéroatomes.

Z₁ répond de préférence également à une telle définition.

Dans des modes de réalisation particuliers de l'invention, Z, et le cas échéant Z1, sont choisis parmi les groupes (VIIIa) à (VIIIf) suivants :

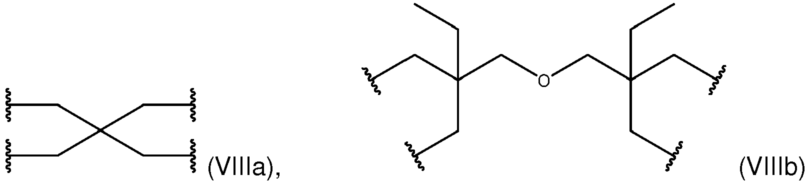

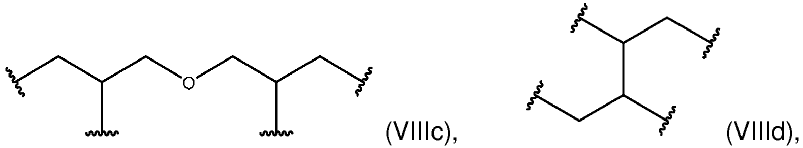

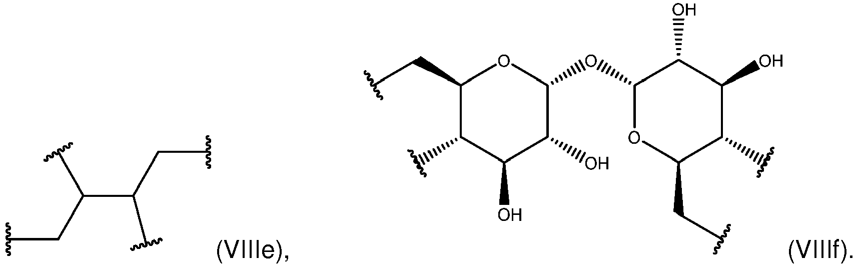

Une telle définition de Z, et le cas échéant de Z1, s'avère notamment tout à fait avantageuse en combinaison avec la caractéristique selon laquelle Y et Y', et le cas échéant Y₁, représentent chacun un groupe de formule générale (VI) ci-dessus.

Le polymère selon l'invention peut en particulier répondre à la formule générale (IV) : 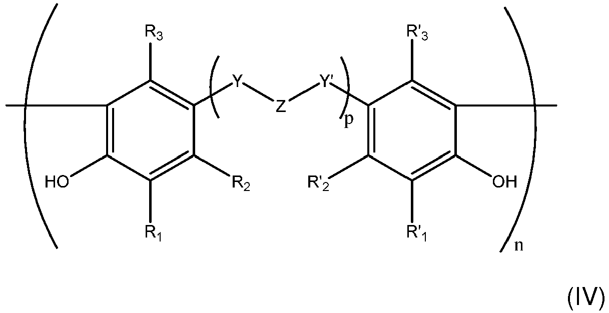 dans laquelle n représente un nombre entier compris entre 2 et 100.

Ce composé peut aussi bien être linéaire que cyclique.

A titre d'exemple, à partir des macrobisphénols biosourcés décrits dans la publication de Pion et al., 2013, on obtient notamment des polymères conformes à l'invention, de formules générales respectives (IVa), (IVb), (IVc) et (IVd) ci-après : 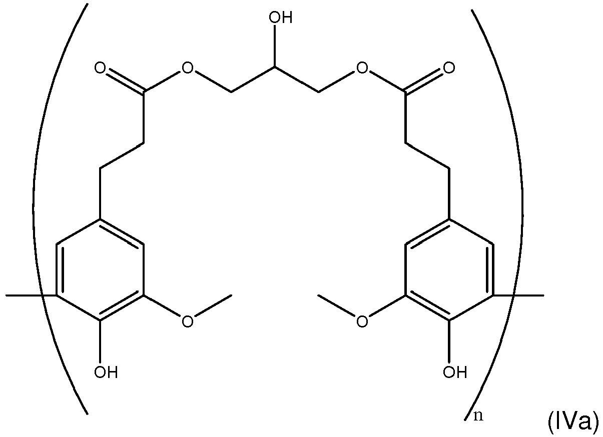 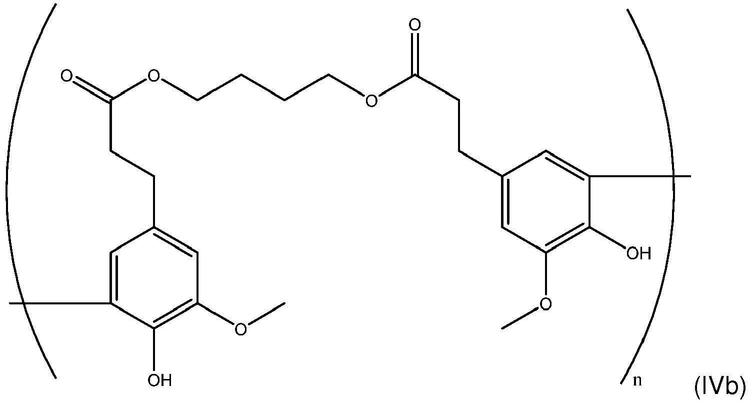 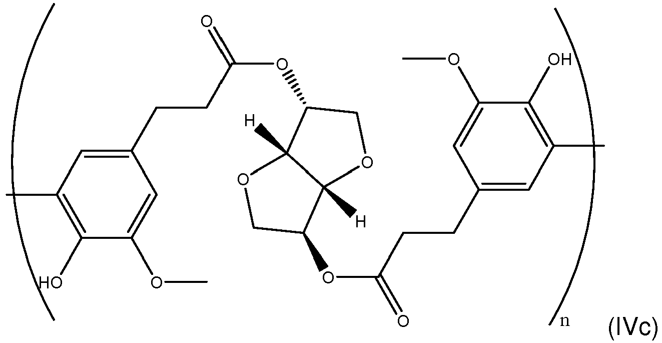 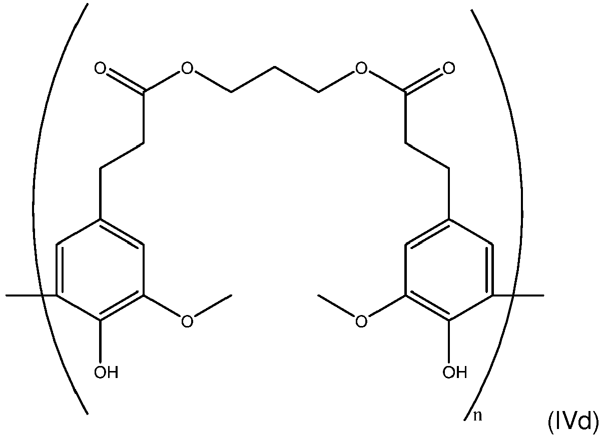 où n représente un nombre entier compris entre 2 et 100.

Ces polymères présentent avantageusement tous une faible toxicité pour les organismes vivants.

Le polymère selon l'invention peut notamment répondre à la formule générale (VII) ci-après : 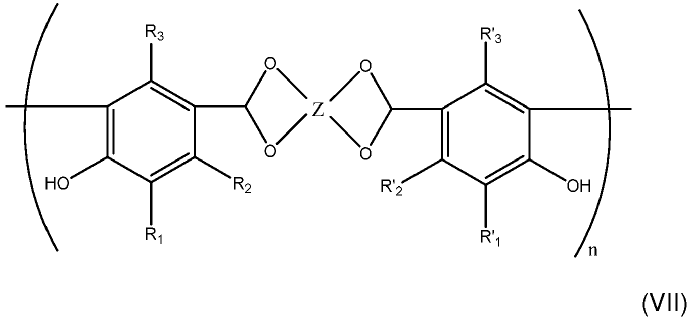 dans laquelle n représente un nombre entier compris entre 2 et 100, et R₁, R₂, R₃, R'₁, R'₂, R'₃ et Z sont tels que définis ci-avant.

Des exemples de tels polymères répondent aux formules générales (VIIa) à (VIIf) ci-après :

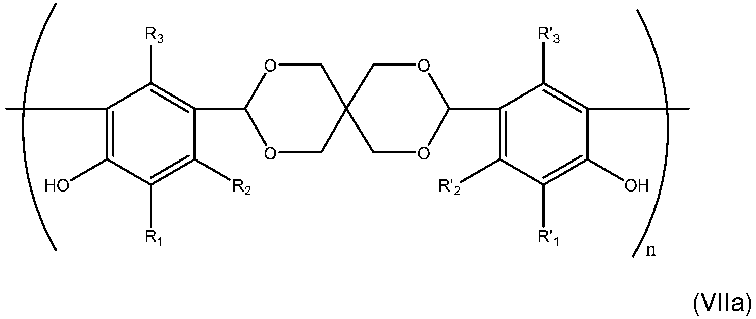

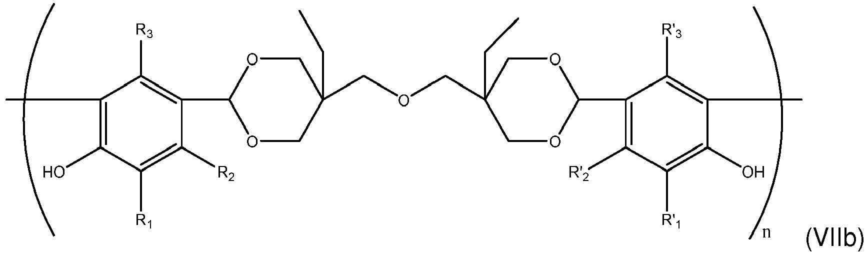

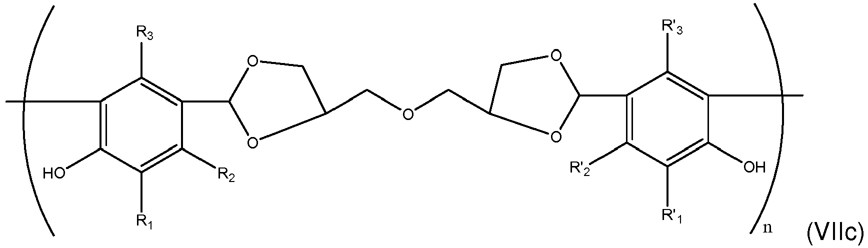

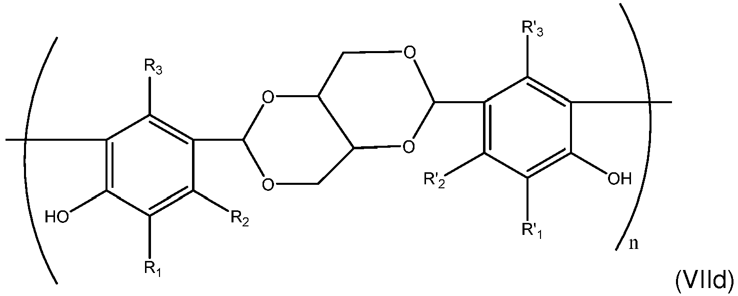

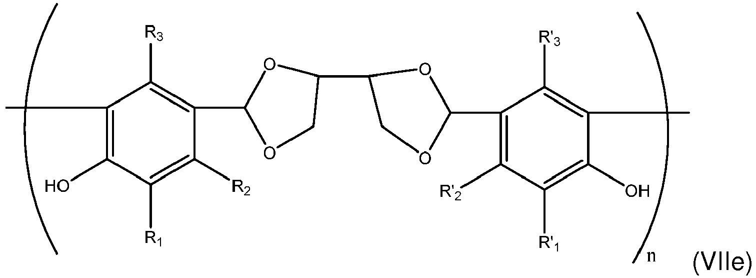

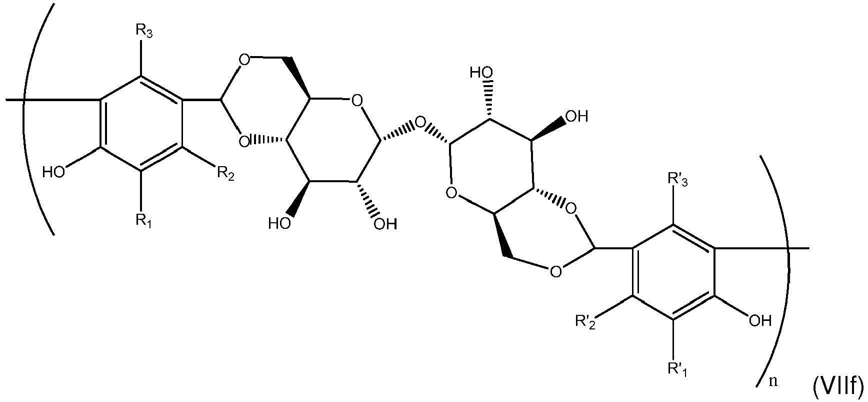

Un autre exemple de polymère conforme à la présente invention répond à la formule (Vllg) ci-après :

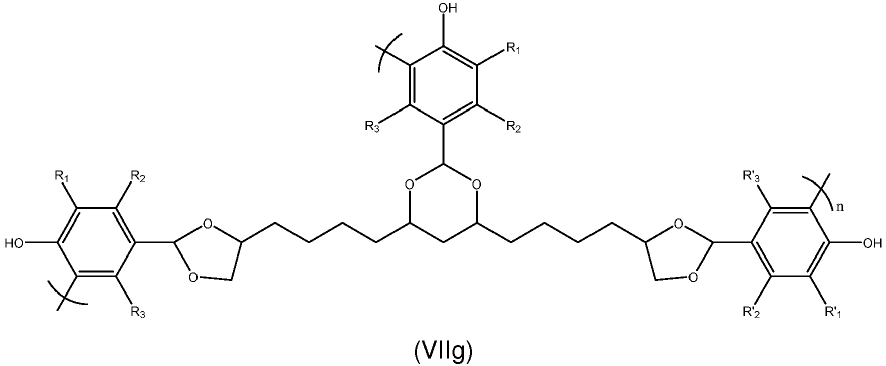

Outre par oligomérisation catalysée par une enzyme de type oxydase, les polymères phénoliques selon l'invention, par exemple les polymères répondant aux formules générales (VII), (VIIa), (VIIb), (VIIc), (VIId), (VIIe), (VIIf) et (VIIg) ci-dessus, peuvent être obtenus par toute autre voie de synthèse, dont la ou les étapes sont classiques en elles-mêmes pour l'homme du métier.

Selon un autre aspect, la présente invention concerne un procédé de synthèse d'un polymère phénolique selon l'invention, qui comprend une étape d'oligomérisation d'un ou plusieurs macropolyphénol(s) répondant chacun à la formule générale (I) ci-dessus, catalysée par une enzyme de type oxydase.

Un tel procédé, qui s'inscrit dans le domaine des biotechnologies blanches, est avantageusement simple et peu onéreux à mettre en oeuvre, en particulier par rapport aux procédés chimiques conventionnels qui nécessiteraient, pour parvenir au même résultat, de nombreuses réactions de protection/déprotection des fonctions phénols réactives du ou des macropolyphénol(s). Il permet en outre notamment de contrôler le degré de polymérisation du ou des macropolyphénol(s) de base, en particulier par un contrôle adéquat du temps de réaction et de la nature et de la quantité du co-solvant. Lorsque le / les macropolyphénol(s) sont d'origine biosourcée, le procédé selon l'invention s'avère de plus tout à fait avantageux d'un point de vue écologique.

Toute enzyme de la famille des oxydases peut être utilisée pour catalyser la réaction. Une peroxydase, telle que la peroxydase de raifort, peut notamment être mise en oeuvre.

Dans des modes de mise en oeuvre préférés de l'invention, l'enzyme est une laccase. Les laccases sont des enzymes bien connues en elles-mêmes, appartenant à la classe des oxydases, et présentes dans les plantes et les champignons. Elles catalysent les oxydations d'un électron des substrats riches en électrons, tels que les substrats phénoliques, en utilisant l'oxygène de l'air en tant qu'oxydant. Les radicaux résultant peuvent alors subir d'autres réactions telles que les dimérisations et les polymérisations.

Les laccases de toute origine, aussi bien végétale, que fongique ou animale, peuvent être mises en oeuvre selon l'invention. Leur principal intérêt est leur simplicité d'utilisation, la réaction pouvant notamment être conduite en réacteur ouvert, et l'absence de besoin de co-oxydants dangereux. Elles peuvent en outre fonctionner dans de larges gammes de température et de pH.

On ne préjugera pas ici du mécanisme de la réaction de couplage oxydatif catalysée par l'enzyme, sous-tendant l'obtention tout à fait avantageuse d'un unique produit de réaction, se caractérisant par un seul type de liaison entre les fragments monomères de base du polymère, laissant en outre libres les fonctions hydroxyles des noyaux phénoliques. On peut cependant supposer que le choix réalisé par les présents inventeurs, de groupements Y, Y' et le cas échéant Y₁ déconjuguants en position para de l'hydroxyle sur le cycle phénolique, participe, avec les groupes particuliers R₁, R'₁, R₂, R'₂, R₃, R'₃, à stabiliser le radical formé par l'oxydase en position 5 du cycle phénolique.

L'étape d'oligomérisation peut être réalisée à partir d'un unique monomère de formule générale (I), ou d'une pluralité de tels monomères, par exemple de deux ou trois macropolyphénols de formule générale (I) différents. Dans ce cas, les différents macropolyphénols peuvent être introduits dans le milieu réactionnel en quantités équivalentes, ou différentes, en fonction du polymère phénolique visé.

En particulier, le mélange de monomères initiaux peut comporter toute combinaison de types de macropolyphénols, par exemple deux ou plus macrobisphénols, un ou plusieurs macrobisphénols et un ou plusieurs macrotrisphénols, etc.

Par ailleurs, lorsqu'au moins un des monomères de départ n'est pas un macrobisphénol, mais par exemple un macrotrisphénol, il se crée dans la chaîne polymère des points de réticulation entre différents fragments macropolyphénols, formant ainsi avantageusement un polymère réticulé tridimensionnel de type résine phénolique.

Dans des modes de mise en oeuvre particuliers de l'invention, l'étape d'oligomérisation est réalisée en solution aqueuse, et dans au moins une des conditions suivantes :
- à température comprise entre 0 et 75 °C, de préférence entre 20 et 60 °C, et préférentiellement à environ 40 °C,
- à pH compris entre 3 et 8.

La solution aqueuse peut en outre comporter de 0 à 80 % en volume d'un solvant organique. Une large gamme de solvants organiques peut notamment être utilisée, en particulier, mais non limitativement, les alcools, les alcanes, les éthers, les solvants aromatiques, les amines, etc.

Dans d'autres modes de mise en oeuvre particuliers de l'invention, l'étape d'oligomérisation est réalisée dans un liquide ionique. Une telle caractéristique s'avère notamment tout à fait avantageuse lorsqu'un ou plusieurs des macropolyphénols de formule générale (I), destinés à entrer dans la constitution du polymère phénolique selon l'invention, sont instables en milieu aqueux.

Tout liquide ionique connu de l'homme du métier peut être mis en oeuvre à cet effet. A titre d'exemples, on peut citer les liquides ioniques suivants :
- [Emim] [EtSO₄] (*1-ethyl-3-methylimidazolium, ethyl sulfate*),
- [Emim] [EtSO₄] (*1-ethyl-3-methylimidazolium, ethyl sulfate*),
- [TMA] [TfO] (*tetramethylammonium trifluoromethanesulfonate*),
- [C₆mim] [AOT] = *1-hexyl-3-methylimidazolium*, *1,4-bis(2-ethylhexoxy)-1,4-dioxo-butane-2-sulfonate (docusate)*),
- [3-butyl-1-[(1*R*,2*S*,5*R*)-(-)-menthoxymethyl]imidazolium] [NTf₂] (*3-butyl-1-[(1R,2S,5R)-(-)-menthoxymethyl]imidazolium, bis(trifluoromethanesulfonyl)imide*),
- [1-[(1*R*,2*S*,5*R*)-(-)-menthoxymethyl]-3-methylpyridinium] [NTf₂] (*1-[(1R,2S,5R)-(-)-menthoxymethyl]-3-methylpyridinium*, *bis(trifluoromethanesuifonyl)imide)*,
- [heptyl[(1*R*,2*S*,5*R*)-(-)-menthoxymethyl]dimeth-ylammonium]-3-methylpyridinium] [NTf₂] (*hepty*/*[(1R,2S,5R)-(-)-menthoxymethy]ammonium*, *bis(trifluoromethanesulfonyl)imide),*
- [decyl[(1*R*,2*S*,5*R*)-(-)-menthoxy-methyl]dimethylammonium] [NTf₂] (*decy*/*[(1R,2S,5R)-(-)-menthoxy-methyl]dimethylammonium*, *bis(trifluoromethanesulfonyl)imide*).

Préférentiellement, une quantité minimale de laccase de 2 unités par millimole de macropolyphénol est mise en oeuvre, pour obtenir un taux de conversion du macropolyphénol supérieure ou égale à 70 %.

Selon un autre aspect, l'invention concerne une composition comportant un polymère selon l'invention, dans un véhicule choisi en fonction de l'application particulière visée, par exemple dans un véhicule cosmétiquement acceptable, ou encore dans une matrice polymère ou composite.

Le polymère selon l'invention peut notamment être incorporé dans diverses solutions, notamment alimentaires, émulsions ou crèmes, en tant qu'agent antioxydant/antiradicalaire et/ou antimicrobien. Il peut autrement être incorporé dans des matrices polymères ou composites, par exemple par extrusion réactive ou par un mélangeur interne, de sorte à conférer à ces dernières des propriétés antioxydantes/antiradicalaires et/ou plastifiantes.

Le polymère selon l'invention peut également être déposé, de manière covalente ou non, sur une surface polymère, composite ou métallique, de sorte à conférer à cette surface des propriétés antioxydantes/antiradicalaires et/ou antimicrobiennes. Ainsi, la présente invention concerne également une pièce dont une surface est revêtue d'une couche formée à base d'un polymère selon l'invention. Cette couche peut être aussi bien continue que discontinue. Le polymère selon l'invention peut en particulier être fixé à la surface de la pièce de manière covalente, notamment par greffage chimique.

Le polymère selon l'invention, en particulier obtenu à partir d'un macrobisphénol, présente un potentiel antioxydant élevé, qui le rend notamment tout à fait adapté pour des applications dans le domaine de la cosmétologie, de l'emballage alimentaire, de la détoxification, de l'agroalimentaire, etc.

Ainsi, selon l'invention, ce polymère peut être utilisé en tant qu'agent antioxydant, pour inhiber l'oxydation d'une substance, par mise en contact du polymère avec cette substance.

En particulier, il a été démontré par les présents inventeurs, par le test bien connu sous le nom de DPPH (pour 1,1-diphényl-2-pycril hydrazil) de mesure de l'activité antioxydante/antiradicalaire des substances, que les polymères linéaires conformes à l'invention, de formules respectives (IVa), (IVb), (IVc) et (IVd), présentent un pouvoir antioxydant supérieur à celui de l'acide férulique.

La présente invention concerne également l'utilisation d'un polymère conforme à l'invention en tant qu'agent plastifiant.

Le polymère selon l'invention trouve également des applications en tant qu'agent chélatant, ou encore en tant qu'agent antimicrobien. En cela, il est notamment tout à fait adapté pour des applications de traitement de surface.

Les caractéristiques et avantages de l'invention apparaîtront plus clairement à la lumière des exemples de mise en oeuvre ci-après, fournis à simple titre illustratif et nullement limitatifs de l'invention, avec l'appui des figures 1 à 14, dans lesquelles :
- la figure 1a montre un chromatographe obtenu par chromatographie d'exclusion stérique pour un polymère B1 formé par un procédé de synthèse conforme à l'invention, avec détection à 250 nm ;
- la figure 1b montre un chromatographe obtenu par chromatographie d'exclusion stérique pour un polymère P1 formé par un procédé de synthèse conforme à l'invention, avec détection à 250 nm ;
- la figure 1c montre un chromatographe obtenu par chromatographie d'exclusion stérique pour un polymère G1 formé par un procédé de synthèse conforme à l'invention, avec détection à 250 nm ;
- la figure 2a montre un spectre de RMN ¹³C d'un polymère B1 formé par un procédé de synthèse conforme à l'invention ;
- la figure 2b montre un spectre de RMN ¹³C d'un polymère P1 formé par un procédé de synthèse conforme à l'invention ;
- la figure 2c montre un spectre de RMN ¹³C d'un polymère G1 formé par un procédé de synthèse conforme à l'invention ;
- la figure 2d montre un spectre de RMN ¹³C d'un polymère I1 formé par un procédé de synthèse conforme à l'invention ;
- la figure 2e montre les déplacements chimiques en RMN ¹³C, en ppm, prédits par simulation par le logiciel ChemBiodrawUltra 13.0.2®, pour différents types de liaison entre motifs phénoliques ;
- la figure 3 montre les résultats obtenus par analyse MALDI-TOF d'un polymère B1 formé par un procédé de synthèse conforme à l'invention ;
- la figure 4 montre l'évolution du taux de conversion du monomère PDF en polymère linéaire par un procédé de synthèse conforme à l'invention, en fonction du % de co-solvant acétate d'éthyle dans la solution réactionnelle aqueuse,
- la figure 5 montre l'évolution du taux de conversion du monomère PDF en polymère linéaire par un procédé de synthèse conforme à l'invention, en fonction du pH de la solution réactionnelle aqueuse, avec de l'acétate d'éthyle en tant que co-solvant ;
- la figure 6 montre l'évolution du taux de conversion du monomère PDF en polymère linéaire par un procédé de synthèse conforme à l'invention, en fonction de la nature du co-solvant dans la solution réactionnelle aqueuse, le % de co-solvant dans cette dernière étant de 30 % (v/v) ;
- la figure 7 montre un graphe représentant, en fonction de la durée de réaction, le taux de conversion du monomère BDF et la masse moléculaire moyenne en nombre (Mn) du polymère linéaire obtenu par un procédé de synthèse conforme à l'invention ;
- la figure 8 montre les spectres obtenus par chromatographie d'exclusion stérique pour des polymères linéaires formés à partir du monomère BDF par des procédés de synthèse conformes à l'invention, mettant en oeuvre différentes concentrations de monomère dans le milieu réactionnel ;
- la figure 9 montre un graphe représentant, en fonction de la température appliquée, les taux de conversion du monomère GDF et les masses moléculaires moyennes en nombre (Mn) des polymères linéaires obtenus par des procédés de synthèse conformes à l'invention mettant en oeuvre, en tant que co-solvant, respectivement de l'acétone et de l'éthanol (EtOH) ;
- la figure 10 montre un graphe représentant, en fonction de la température appliquée, les taux de conversion du monomère IDF et les masses moléculaires en nombre (Mn) des polymères linéaires obtenus par des procédés de synthèse conformes à l'invention mettant en oeuvre en tant que co-solvant respectivement de l'acétone et de l'éthanol (EtOH) ;
- la figure 11 montre un graphe représentant, en fonction de la charge de laccase utilisée, pour chacun des monomères respectifs GDF et IDF, le taux de conversion et la masse moléculaire moyenne en nombre (Mn) du polymère linéaire obtenu par un procédé de synthèse conforme à l'invention ;
- la figure 12 montre un graphe représentant, pour des macrobisphénols répondant à la formule (I), nommés respectivement IDF, GDF, BDF et PDF, le % d'activité antagoniste de l'activité de l'oestradiol (E2) à 10 nM, en fonction de la concentration du macrobisphénol ;
- la figure 13 montre un spectre de RMN ¹³C d'un polymère formé par un procédé de synthèse conforme à l'invention, à partir de macropolyphénols BDF et IDF ;
- et la figure 14 montre un spectre de RMN 2D (corrélation ¹H/¹³C) du polymère de la figure 13.

### A/ Synthèse des polymères phénoliques

### A.1/ Monomères de macrobisphénols

Les monomères (Ia) (glycérol-3-di(dihydroférulate), dit GDF), (Ib) (butane-1,4-di(dihydroférulate), dit BDF), (Ic) (isosorbide-2,5-(dihydroférulate), dit IDF) et (Id) (propane-1,3-di(dihydroférulate), dit PDF), suivants sont utilisés pour la synthèse de polymères phénoliques conformes à l'invention :

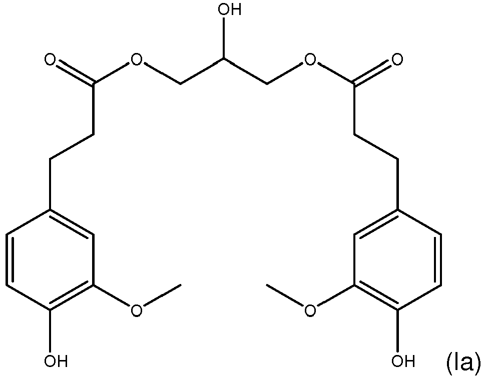

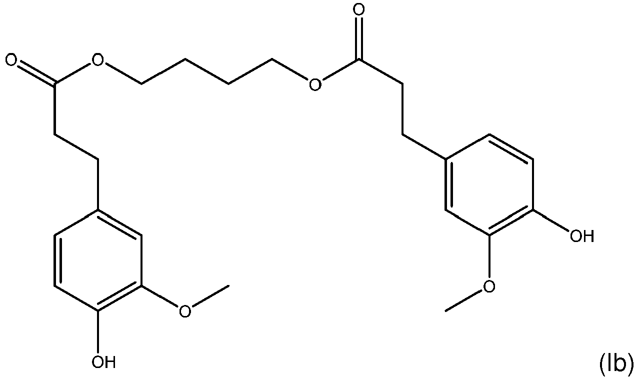

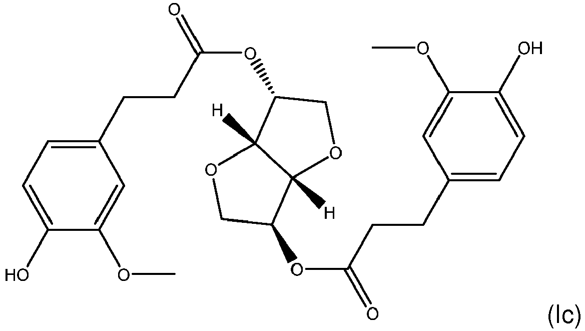

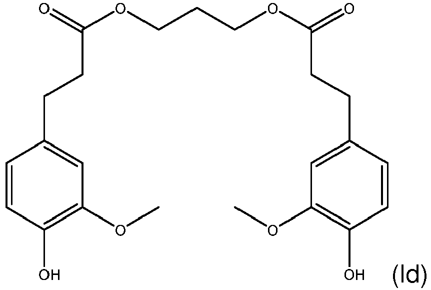

Le macrotrisphénol de formule générale (le) ci-après est également mis en oeuvre en tant que monomère pour la synthèse de polymères phénoliques conformes à l'invention :

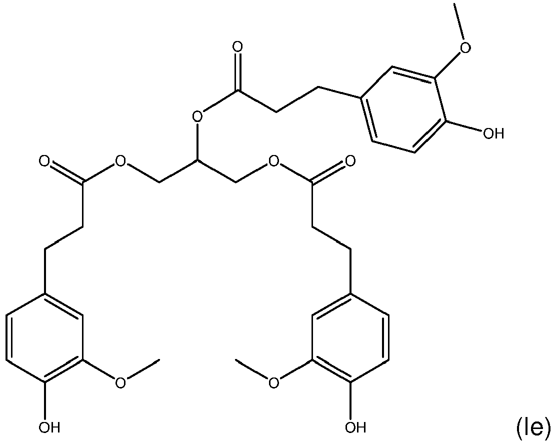

Ces monomères sont préparés selon le procédé décrit dans la publication de Pion et al., 2013, au moyen d'une lipase B, à partir d'acide férulique et de polyols.

### A.2/ Protocole de synthèse des polymères

Les polymères sont synthétisés par oligomérisation des monomères ci-avant, au moyen d'une laccase, qui forme un radical sur chaque noyau phénol, permettant ainsi l'oligomérisation par un couplage « radical-radical ».

Le mode opératoire général est le suivant.

Le macrobisphénol ou le macrotrisphénol est pesé dans un ballon puis dissous dans le co-solvant choisi avant d'y ajouter de l'eau, éventuellement tamponnée pour obtenir le pH souhaité, puis la laccase de *Trametes versicolor,* à une charge choisie dissoute dans l'eau, éventuellement tamponnée pour obtenir le pH souhaité. Le milieu est vigoureusement agité magnétiquement pendant un temps choisi, à température choisie et à l'air libre ou sous atmosphère d'oxygène.

Lorsqu'un solide est présent en fin de réaction, il est récupéré puis séché. Autrement, le milieu réactionnel est repris dans un volume de dichlorométhane ou d'acétate d'éthyle, égal à 3 fois le volume réactionnel, pour en extraire les composés organiques, c'est-à-dire le polymère formé et, le cas échéant, le macrobisphénol ou le macrotrisphénol n'ayant pas réagi. La phase organique ainsi obtenue est séchée en présence de sulfate de magnésium anhydre (MgSO₄), filtrée et concentrée sous vide.

On obtient ainsi, en fonction du monomère de départ, les polymères respectifs (IVa), (IVb), (IVc) et (IVd) décrits ci-avant, ainsi, à partir du macrotrisphénol de formule (le), qu'un polymère ramifié comportant exclusivement des liaisons de type biaryle 5-5 entre les fragments macrotrisphénol de base.

Différentes expériences ont ainsi été réalisées avec, pour chaque monomère, diverses combinaisons des conditions réactionnelles suivantes :
- co-solvant choisi parmi : méthanol, éthanol, isopropanol, tert-butanol, butanol, alcool benzylique, éthylène glycol, hexane, heptane, dichlorométhane, 1,4-dioxane, tétrahydrofurane, acétone, 4-méthyl-2-pentanone, acétate d'éthyle (AcOEt), diéthyléther, 1,2-diméthyléther, diéthylsuccinate, diméthylformamide, chloroforme, pyridine, benzène, o-dichlorobenzène ou acétonitrile ;
- solution aqueuse, par exemple d'eau Milli-Q®, pure ou tamponnée avec un tampon choisi parmi les tampons phosphate et les tampons acétate de sodium, pour un pH compris entre 2 et 7, plus précisément de 2,3 ; 2,9 ; 3,7 ; 3,9 ; 4,2 ou 5,6 ;
- % de co-solvant dans la solution aqueuse compris entre 0 et 100 % en volume, plus particulièrement de 0, 20, 25, 29, 30, 40, 45, 60, 80 ou 100 % (v/v) ;
- concentration en macrobisphénol dans la solution comprise entre 3 et 50 g/L, plus particulièrement égale à 3,2 ; 6,4 ; 6,5 ; 13 ; 16,6 ; 20 ; 25 ; 27 ; 33 ; 33,33 ; 43 ; 45 ou 50 g/l ;
- charge de laccase comprise entre 2 et 1000 unités par millimole de macrobisphénol, plus particulièrement égale à 2, 10, 50, 100, 200 ou 1000 u/mmol,
- température comprise entre 0 et 80 °C, plus particulièrement égale à 5, 20, 40, 50, 60 ou 80 °C,
- durée comprise entre 8 et 120 h, plus particulièrement égale à 8, 18, 24, 48, 72, 96 ou 120 h.

A titre d'exemples particuliers, les polymères suivants sont formés dans les conditions indiquées dans le tableau 1 ci-après.

**Tableau 1 - Conditions réactionnelles d'obtention de polymères conformes à l'invention**

| Polymère | Monomère | Solvant (% v/v) | pH | Concentration en monomère (g/l) | Laccase (u/mmol) | Temp. (°C) | Durée (h) |
|---|---|---|---|---|---|---|---|
| P1 | PDF | EtOH (30) | - | 20 | 100 | 40 | 120 |
| B1 | BDF | Acétone (45) | 4,2 | 6,5 | 1000 | 20 | 120 |
| G1 | GDF | Acétone (25) | 4,2 | 25 | 10 | 40 | 96 |
| PDF 1000 | PDF | AcOEt (40) | 3,7 | 33,33 | 50 | 20 | 72 |
| PDF 1500 | PDF | AcOEt (20) | 3,7 | 33,33 | 50 | 20 | 72 |

### A.3/ Analyse des produits obtenus en fonction des paramètres opératoires

Pour chaque expérience, une analyse de chromatographie par exclusion stérique (HPSEC) est réalisée afin de déterminer le taux de conversion, la courbe de distribution des masses moléculaires, et d'évaluer les masses moléculaires moyennes des produits obtenus, au moyen d'un dispositif comportant une pompe Gilson 305, un injecteur UltiMate® 3000 ACC de Dionex, une colonne PLgel 5µm 100, 600 x 7.5 mm et un détecteur UV PDA 3000 de Dionex.

Le vecteur utilisé est le tétrahydrofurane à un débit de 1 mL/min, et la détection est réalisée à 250 nm.

Le calibrage du dispositif d'analyse est réalisé au moyen d'étalons Igepal®.

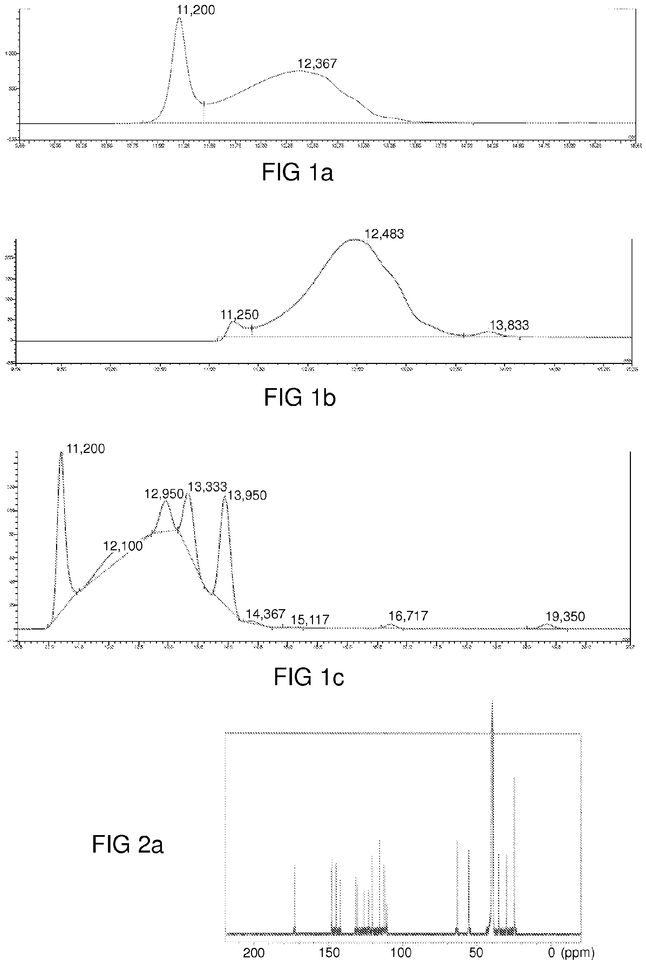

A titre d'exemple, les spectres obtenus pour les polymères conformes à la présente invention B1, formé à partir du monomère BDF, P1, formé à partir du monomère PDF, et G1, formé à partir du monomère GDF, comme indiqué ci-avant, sont montrés respectivement sur les figures 1a, 1b, 1c. On y observe, pour chaque polymère conforme à l'invention, une conversion du monomère quasi-totale, une bonne dispersité du signal HPSEC, et un degré de polymérisation relativement élevé. Chacun des polymères selon l'invention est largement majoritaire dans le mélange correspondant obtenu.

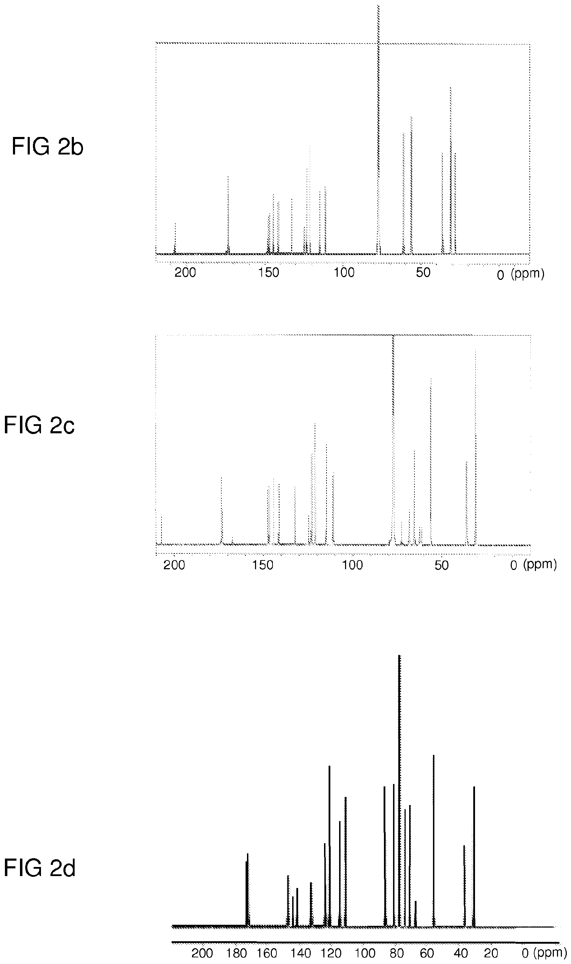

Les spectres de RMN ¹³C de chacun de ces polymères conformes à l'invention ont également été réalisés (DMSO-d₆ ou CDCl₃), et sont montrés respectivement sur les figures 2a, 2b et 2c pour les polymères B1, P1 et G1.

Un spectre de RMN ¹³C a également été réalisé pour un polymère, nommé I1, obtenu conformément à l'invention avec le macrobisphénol IDF en tant que monomère. Ce spectre est montré sur la figure 2d.

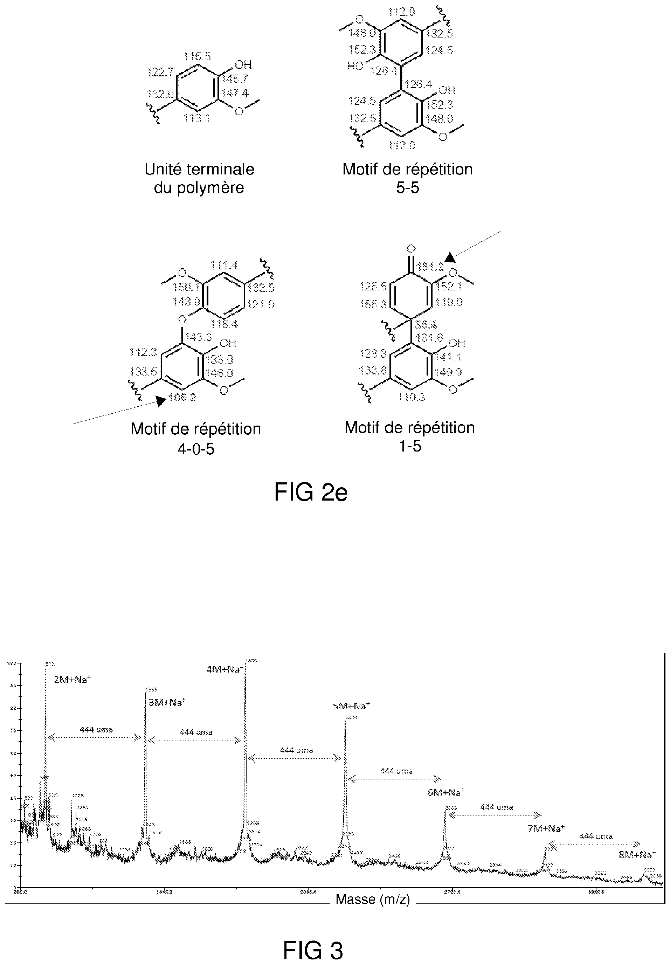

L'ensemble de ces spectres ont été comparés aux déplacements chimiques prédits par simulation par le logiciel ChemBiodrawUltra 13.0.2®, pour différents types de liaison entre les motifs phénoliques, qui sont montrés sur la figure 2e. Cette comparaison démontre clairement que les liaisons dans les polymères B1, P1 et G1 sont bien du type biaryle 5-5. En effet, il n'est présent sur les spectres de ces polymères aucun pic caractéristique d'une liaison 1-5, à 181,2 ppm, et aucun pic caractéristique d'une liaison 4-*O*-5, à 106,2 ppm.

Des analyses MALDI-TOF ont en outre été réalisées sur les polymères conformes à l'invention B1, P1 et G1. Le résultat obtenu pour le polymère B1 est montré sur la figure 3. On y observe pour ce polymère un enchaînement régulier, et un degré de polymérisation maximal égal à 8. Ce résultat est représentatif de celui obtenu pour les polymères P et G1, ainsi que pour tous les polymères obtenus par un procédé de synthèse conforme à l'invention.

L'ensemble des spectres obtenus montre que pour toutes les combinaisons des paramètres opératoires, on obtient très majoritairement un polymère linéaire et homogène, caractérisé par un seul type de liaison biaryle 5-5 entre les monomères macrobisphénols.

A partir des spectres obtenus, l'influence des différents paramètres opératoires sur le polymère formé a été étudiée.

### Influence du solvant

Le taux de conversion du monomère de base, en polymère conforme à l'invention a été évalué pour le monomère PDF, en faisant varier respectivement le % de co-solvant, le pH du milieu réactionnel et la nature du co-solvant.

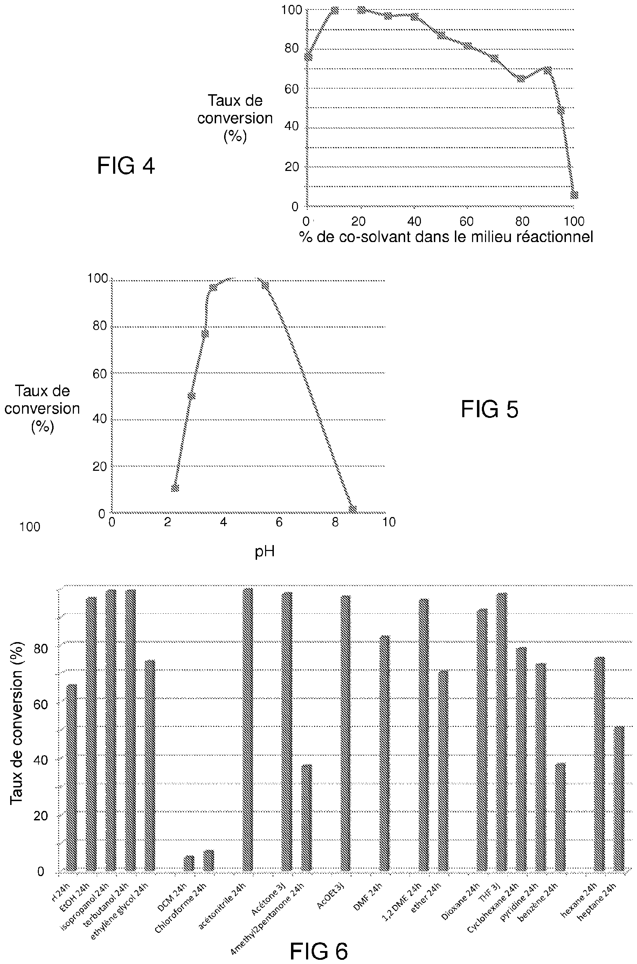

Les résultats sont montrés respectivement sur la figure 4 (variation du % de co-solvant, les conditions opératoires étant les suivantes : co-solvant AcOEt, température 20 °C, pH 3,7, concentration en monomère 1 g/30mL), la figure 5 (variation du pH, les conditions opératoires étant les suivantes : co-solvant AcOEt à 30 % v/v, température 20 °C) et la figure 6 (variation de la nature du co-solvant, les conditions opératoires étant les suivantes : 30 % v/v de co-solvant, température 20 °C, pH 3,7, durée indiquée sur le graphe après le nom du co-solvant). Ces résultats sont représentatifs de ceux obtenus pour tous les autres monomères de départ, et toutes les combinaisons de conditions opératoires. Ils démontrent que le procédé d'oligomérisation catalysé par la laccase peut avantageusement être mis en oeuvre avec une large gamme de co-solvants, dans une large gamme de pH, et aussi bien en milieu aqueux strict qu'en milieu mixte eau/co-solvant, et ce jusqu'à des proportions élevées de co-solvant.

### Influence de la durée de réaction

L'influence de la durée de réaction a été évaluée à partir du monomère BDF, dans les conditions opératoires suivantes : température de 20 °C, charge de laccase de 50 u/mmol, pH de 4,2, co-solvant acétone à 60 % (v/v), concentration initiale de 6,4 g/L de monomère. Des durées de réaction comprises entre 8 et 120 h ont été testées.

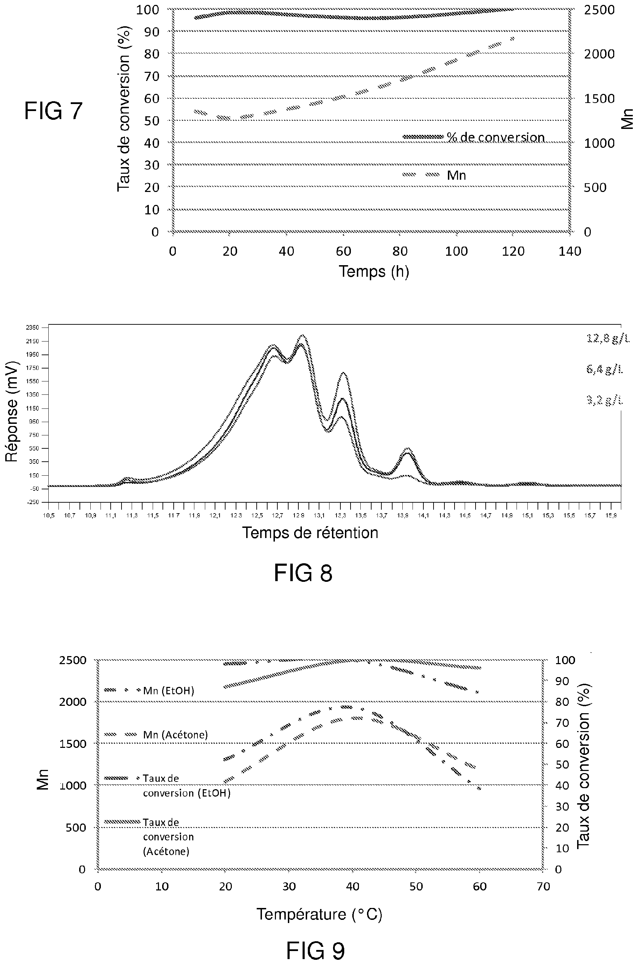

Les résultats obtenus, en termes de taux de conversion du monomère et de masse moléculaire moyenne en nombre, en fonction de la durée de réaction, sont montrés sur la figure 7. On y observe que le taux de conversion maximal est très rapidement atteint au cours de la réaction, alors que la masse moléculaire en nombre augmente de manière continue avec la durée de réaction. Ceci démontre que la dimérisation du monomère BDF est rapide, l'oligomérisation mettant un peu plus de temps à se réaliser. Le contrôle du temps de réaction permet ainsi de contrôler le degré de polymérisation.

Ces résultats sont représentatifs de ceux obtenus pour tous les autres monomères de départ, et toutes les combinaisons de conditions opératoires.

### Influence de la dilution

L'influence de la dilution du monomère dans le milieu réactionnel a été analysée pour le monomère BDF, dans les conditions opératoires suivantes : température de 20 °C, charge de laccase de 50 u/mmol, pH de 4,2, co-solvant acétone à 60 % (v/v), durée de réaction de 5 jours. Des concentrations initiales en monomère de 3,2, 6,4 et 12,8 g/L ont été testées.

Pour chacune des réactions, les spectres obtenus par chromatographie d'exclusion stérique sont montrés sur la figure 8. On y observe que la dilution a une influence négligeable sur le produit réactionnel obtenu. Ces résultats sont représentatifs de ceux obtenus pour tous les autres monomères de départ, et toutes les combinaisons de conditions opératoires.

### Influence de la température

L'influence de la température a été analysée à partir des monomères respectivement GDF et IDF, dans les conditions opératoires suivantes : concentration de monomère de 28 g/L, charge de laccase de 50 u/mmol, pH de 4,2, co-solvant acétone ou éthanol (EtOH) à 30 % (v/v), durée de réaction de 5 jours. Des températures comprises entre 20 et 80 °C ont été testées.

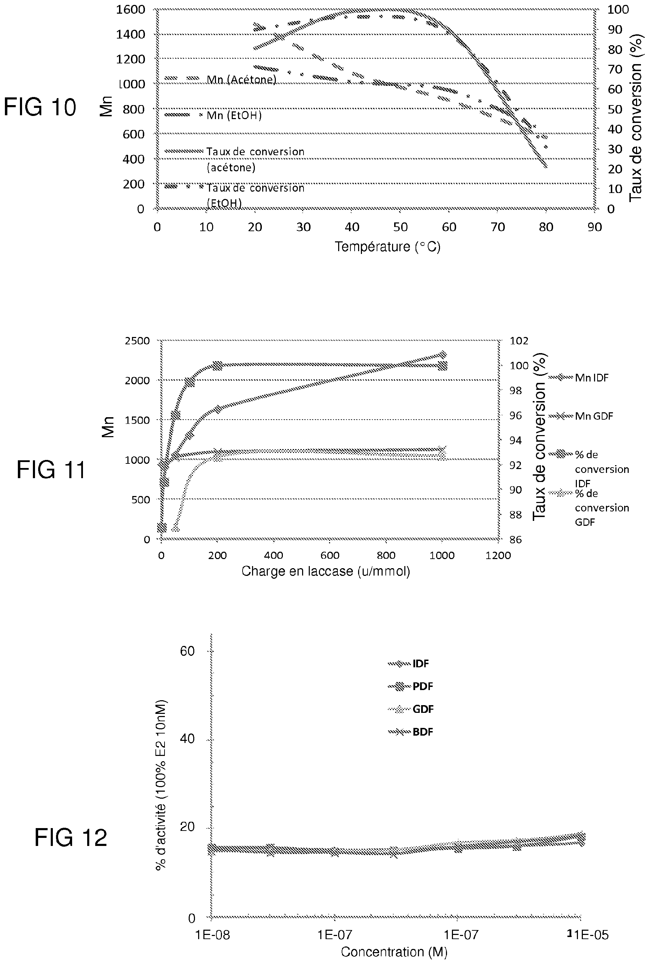

Les résultats obtenus, en termes de taux de conversion du monomère et de masse moléculaire moyenne en nombre (Mn), en fonction de la température et pour chaque monomère et chaque solvant, sont montrés sur la figure 9 pour le monomère GDF et sur la figure 10 pour le monomère IDF. On y observe que la réaction de polymérisation se produit pour des températures jusqu'à environ 75 °C, avec une plage optimale de température comprise entre 20 et 60 °C, et une valeur optimale de 40 °C environ.

Ces résultats sont représentatifs de ceux obtenus pour tous les autres monomères de départ, et toutes les combinaisons de conditions opératoires.

### Influence de la charge en laccase

L'influence de la température a été analysée à partir des monomères respectivement GDF et IDF, dans les conditions opératoires suivantes : concentration de monomère de 28 g/L, pH de 4,2, co-solvant acétone à 30 % (v/v), durée de réaction de 5 jours, température de 20 °C. Des charges de laccase comprises entre 0 et 1100 u/mmol ont été testées.

Les résultats obtenus, en termes de taux de conversion du monomère et de masse moléculaire moyenne en nombre (Mn), en fonction de la charge en laccase et pour chaque monomère, sont montrés sur la figure 11. On y observe que la population des pics à haut poids moléculaire augmente rapidement avec la charge en enzyme, au moins pour l'IDF. La quantité minimale de laccase nécessaire pour un taux de conversion supérieur ou égal à 70 % est de 2 u/mmol de monomère macrobisphénol.

Ces résultats sont représentatifs de ceux obtenus pour tous les autres monomères de départ, et toutes les combinaisons de conditions opératoires.

### A.4/ Synthèse d'un copolymère conforme à l'invention à partir de macrobisphénols

Un copolymère conforme à l'invention a été préparé à partir des macrobisphénols BDF et IDF selon le mode opératoire suivant.

Les macrobisphénols BDF (0,5 g) et IDF (0,5 g) sont pesés dans un ballon puis dissous dans 20 mL d'acétonitrile avant d'y ajouter de l'eau milli-Q®, puis la laccase de *Trametes versicolor* (15,6 mg ; 100 u/mmol_{substrat}) dissoute dans l'eau, le ratio volumique final co-solvant/eau milli-Q® étant de 3/7. Le milieu est vigoureusement agité magnétiquement pendant 24 heures, à température ambiante et à l'air libre. Après 24 heures, une huile jaune s'est formée au fond du ballon.

Le milieu réactionnel est repris dans un volume de dichlorométhane ou d'acétate d'éthyle, égal à 3 fois le volume réactionnel, pour en extraire les composés organiques (oligomères formés et réactifs de départ n'ayant pas réagi). La phase organique ainsi obtenue est séchée en présence de sulfate de magnésium anhydre (MgSO₄), filtrée et concentrée sous vide. On obtient un solide beige.

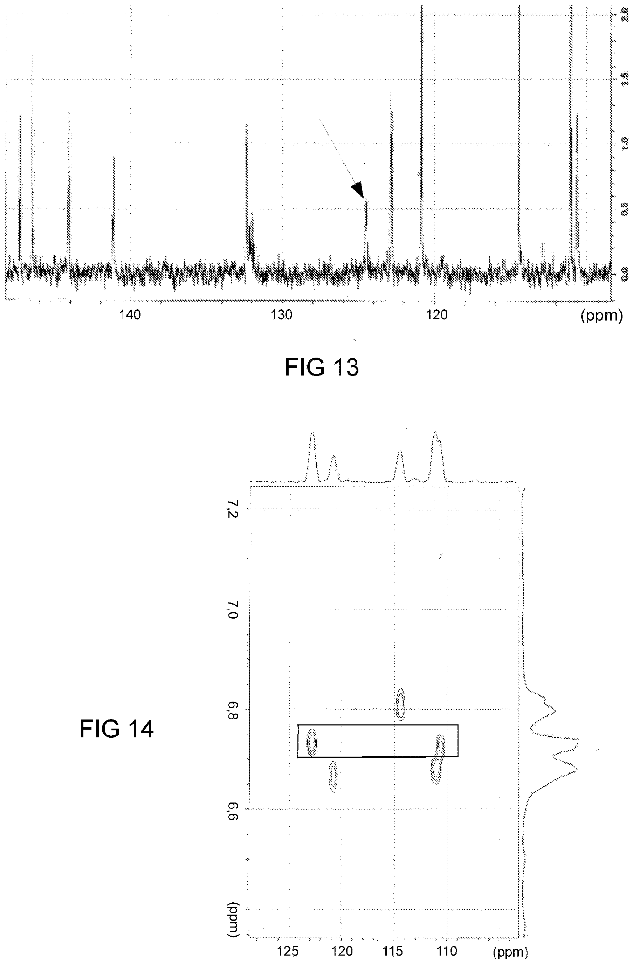

Le spectre de RMN ¹³C du copolymère ainsi obtenu a été réalisé (DMSO-d₆ ou CDCl₃), et est montré sur la figure 13. On y observe un pic à 124,47 ppm, indiqué par une flèche sur la figure, caractéristique de liaisons biaryle 5-5.

Il a également été réalisé une analyse par spectrométrie RMN à deux dimensions (corrélation ¹ H/¹³C). Le spectre obtenu est montré sur la figure 14. Il est caractéristique de polymères phénoliques dont les liaisons biaryle sont uniquement du type 5-5. On observe en particulier, dans l'encadré, les deux signaux représentatifs des deux CH phénoliques des cycles aromatiques formant les liaisons biaryle 5-5 (correspondant au motif de répétition 5-5 montré sur la figure 2e).

Ces résultats d'analyse confirment la formation d'un copolymère à liaisons biaryle exclusivement 5-5.

### A.5/ Synthèse d'un copolymère conforme à l'invention à partir d'un macrobisphénol et d'un macrotrisphénol

Un copolymère conforme à l'invention a été préparé à partir du macrobisphénol PDF et du macrotrisphénol GTF selon le mode opératoire suivant.

Le macrobisphénol PDF (0,5 g) et le macrotrisphénol GTF (0,5 g) sont pesés dans un ballon puis dissous dans 10 mL d'acétonitrile avant d'y ajouter de l'eau milli-Q®, puis la laccase de *Trametes versicolor* (15,6 mg ; 100 u/mmol_{substrat}) dissoute dans l'eau, le ratio volumique final co-solvant/eau milli-Q® étant de 3/7. Le milieu est vigoureusement agité magnétiquement pendant 24 heures, à température ambiante et à l'air libre. Après 24 heures, le milieu réactionnel est devenu marron foncé.

Le milieu réactionnel est repris dans un volume de dichlorométhane ou d'acétate d'éthyle, égal à 3 fois le volume réactionnel, pour en extraire les composés organiques (oligomères formés et réactifs de départ n'ayant pas réagi). La phase organique ainsi obtenue est séchée en présence de sulfate de magnésium anhydre (MgSO₄), filtrée et concentrée sous vide. On obtient un solide marron.

### B/ Analyse du pouvoir antioxydant des polymères phénoliques

Le pouvoir antioxydant des polymères phénoliques conformes à l'invention PDF 1000 et PDF 1500, a été évalué par le test DPPH, selon le protocole décrit dans la publication de Brand-Williams et al., 1995 (Food Sci. Technol-Leb, 28, 25).

A titre d'exemples comparatifs, le pouvoir antioxydant de l'acide férulique, et celui des composés bien connus pour leur pouvoir antioxydant : butylhydroxyl anisol (BHA), butylhydroxytoluène (BHT), acide gallique et acide gentisique, ont été testés dans des conditions équivalentes.

Pour chaque substance à analyser, des concentrations entre 0,12 et 0,00125 x10⁻³mol/L, dans l'éthanol (77 µl) en tant que solvant, ont été testées en présence de 3 mL d'une solution dans l'éthanol de DPPH (soit une concentration de DPPH de 6x10⁻⁵ mol/L). Pour chaque échantillon, l'absorbance a été mesurée en fonction du temps, et le % de DPPH restant en solution a été calculé.

Les résultats obtenus, après 435 min de réaction, c'est-à-dire jusqu'à atteindre une valeur stable correspondant à un plateau, sont montrés dans le tableau 2 ci-après.

**Tableau 2 - Valeurs obtenues par un test DPPH pour des polymères phénoliques conformes à l'invention**

| Polymère | Concentration (x10⁻³ mol/L) | Absorbance optique du DPPH | % de DPPH restant |
|---|---|---|---|
| PDF 1000 | 0,03 | 0,5 | 13,88 |
| | 0,01 | 0,16 | 22,71 |
| | 0,005 | 0,083 | 46,53 |
| | 0,0025 | 0,042 | 70,19 |
| | 0,00125 | 0,021 | 85,33 |
| PDF 1500 | 0,03 | 0,5 | 13,56 |
| | 0,01 | 0,16 | 16,88 |
| | 0,005 | 0,083 | 33,28 |
| | 0,0025 | 0,042 | 59,46 |
| | 0,00125 | 0,021 | 80,44 |

On déduit des données obtenues la concentration de chaque composé permettant de faire diminuer de 50 % la quantité initiale de DPPH (EC50). Cette concentration est indiquée dans le tableau 3 ci-après.

**Tableau 3 - Concentrations EC50 mesurées par un test DPPH**

| Composé | PDF 1000 | PDF 1500 | Acide férulique | BHA | BHT | Acide gallique | Acide gentisique |
|---|---|---|---|---|---|---|---|
| EC50 | 0,0865 | 0,069 | 0,38 | 0,24 | 0,24 | 0,08 | 0,09 |

Ces résultats montrent clairement que les composés polymères phénoliques conformes à l'invention PDF 1000 et PDF 1500 présentent un pouvoir antioxydant supérieur non seulement à celui de l'acide férulique, mais également à celui des antioxydants couramment utilisés que sont le BHA, le BHT, l'acide gallique et l'acide gentisique.

### C/ Test de toxicité des polymères phénoliques

Un essai de toxicité vis-à-vis des récepteurs oestrogéniques a été réalisé pour les macrobisphénols IDF, PDF, GDF et BDF, selon la méthode exposée dans la publication de Molina-Molina et al., 2008 (Toxicol. Appl. Pharmacol., doi :10.1016/j.taap.2008.07.017), sur la lignée cellulaire HELN-ERα, décrite dans la publication de Escande et al., 2006 (Biochemical Pharmacology, 71, 1459-1469).

Les résultats obtenus, exprimés en % d'activité antagoniste de l'activité de l'oestradiol (E2) à 10 nM, en fonction de la concentration de monomère, sont montrés sur la figure 12. On y observe que les macrobisphénols IDF, PDF, GDF et BDF n'interagissent que très peu avec les récepteurs oestrogéniques. En comparaison, la même expérience réalisée pour le bisphénol A disponible dans le commerce, montre que ce dernier présente un pourcentage d'activité, à 10⁻⁶ M, de 45 %, et, à 10⁻⁵ M, de 60 %. Les macrobisphénols IDF, PDF, GDF et BDF présentent de ce fait une action bien moindre que le bisphénol A, vis-à-vis des récepteurs oestrogéniques.

On peut raisonnablement en déduire qu'il en est de même pour les polymères conformes à l'invention, dont ces macrobisphénols constituent les monomères de base.

## Revendications

1. Polymère phénolique susceptible d'être obtenu par oligomérisation catalysée par une enzyme de type oxydase, d'un ou de plusieurs macropolyphénol(s) répondant chacun à la formule générale (I) : 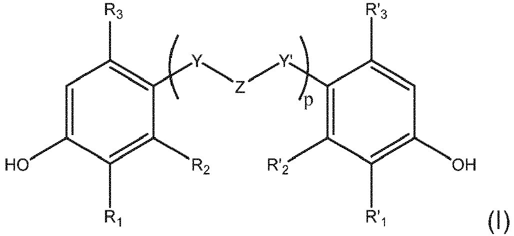 dans laquelle :
p représente un nombre entier compris entre 1 et 30,
R₁, R'₁, R₂, R'₂, R₃ et R'₃, identiques ou différents, représentent chacun un atome d'hydrogène, un atome de chlore, un atome de brome, un atome d'iode, un atome de fluor, un groupe alkyle, benzyle, Xalkyle, le cas échéant substitué, Xbenzyle, le cas échéant substitué, Xacyle, B(OR')₂, NHR', NO₂, SR'O ou SO₂R',
où X représente N, O, S ou P
et R' représente un groupe alkyle ou un groupe aryle,
R₁ et R'₁ ne représentant pas un atome d'hydrogène,
Y et Y', identiques ou différents, représentent chacun :
soit, un atome d'oxygène, un atome de soufre ou un groupe déconjuguant ne comportant ni noyau époxyde, ni noyau aziridine, ni groupement phénol qui ne soit pas substitué sur tous ses atomes de carbone,
soit, un groupe répondant à la formule (II) : 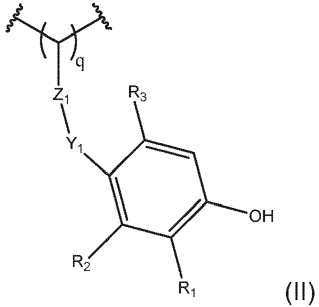
dans laquelle :
q représente un nombre entier compris entre 1 et 8,
Y₁ représente un atome d'oxygène, un atome de soufre ou un groupe déconjuguant ne comportant ni noyau époxyde, ni noyau aziridine, ni groupement phénol qui ne soit pas substitué sur tous ses atomes de carbone,
et Z₁ représente un hétéroatome ou un groupe espaceur ne comportant ni noyau époxyde, ni noyau aziridine, ni groupement phénol qui ne soit pas substitué sur tous ses atomes de carbone, ni groupe alkényle, ni groupe alkynyle,
et Z représente :
soit, un hétéroatome ou un groupe espaceur ne comportant ni noyau époxyde, ni noyau aziridine, ni groupement phénol qui ne soit pas substitué sur tous ses atomes de carbone, ni groupe alkényle, ni groupe alkynyle,
soit, un groupe répondant à la formule (III) : 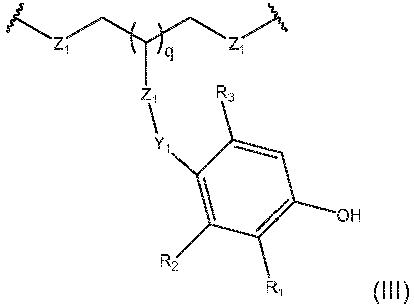 dans laquelle q représente un nombre entier compris entre 1 et 8, les liaisons entre les fragments macropolyphénol de formule générale (I) au sein dudit polymère étant exclusivement des liaisons biaryle 5-5.

2. Polymère selon la revendication 1, dans lequel R₁ et/ou R'₁ représente(nt) un radical hydrocarboné saturé, linéaire ou ramifié, comportant de 1 à 5 atomes de carbone, ou un groupe OR₄, où R₄ représente un radical hydrocarboné saturé, linéaire ou ramifié, comportant de 1 à 5 atomes de carbone.

3. Polymère selon l'une quelconque des revendications 1 à 2, dans lequel R₂, R₃, R'₂ et/ou R'₃ représente(nt) un atome d'hydrogène.

4. Polymère selon l'une quelconque des revendications 1 à 3, dans lequel Y et/ou Y' représente(nt) un groupe de formule générale (V) :
(CH₂)ₘ-X'- (V)
dans laquelle :
m est compris entre 1 et 5,
et X' représente un atome d'oxygène ou un atome de soufre ou un groupe choisi parmi les groupes : 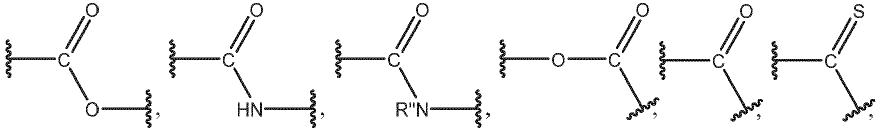 NR", NH ou SO₂, où R" représente un groupe alkyle ou un groupe aryle.

5. Polymère selon l'une quelconque des revendications 1 à 3, dans lequel Y et Y', et le cas échéant Y₁, représentent chacun un groupe de formule générale (VI) :

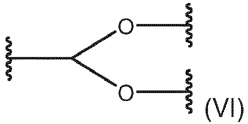

6. Polymère selon l'une quelconque des revendications 1 à 5, dans lequel Z représente un groupe hydrocarboné saturé, linéaire ou ramifié, le cas échéant substitué, comportant de 1 à 6 atomes de carbone, pouvant comporter un ou plusieurs hétéroatomes, ou un groupe hydrocarboné cyclique saturé, le cas échéant substitué, comportant de 1 à 6 atomes de carbone, pouvant comporter un seul cycle ou plusieurs cycles condensés, et pouvant comporter un ou plusieurs hétéroatomes.

7. Polymère selon l'une quelconque des revendications 1 à 6, répondant à la formule générale (IV) : 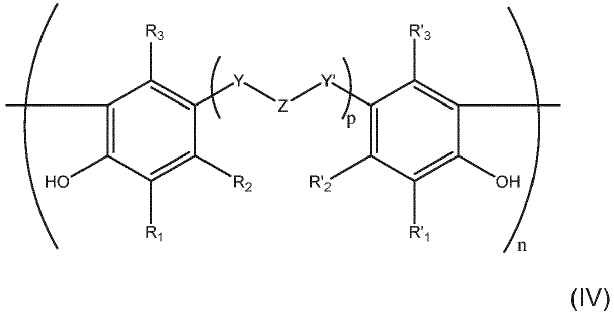 dans laquelle n représente un nombre entier compris entre 2 et 100.

8. Polymère selon l'une quelconque des revendications 1 à 7, répondant à l'une des formules (IVa), (IVb), (IVc) ou (IVd) : 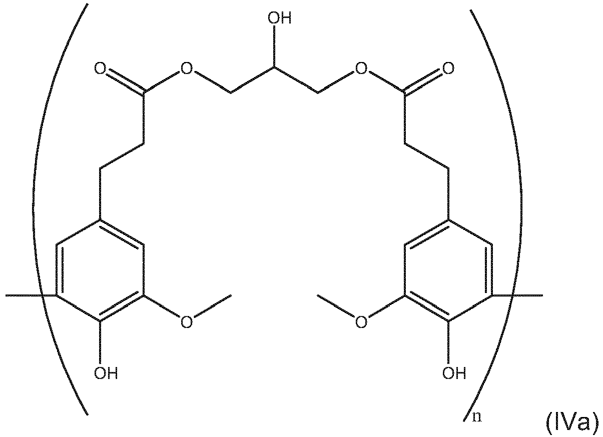 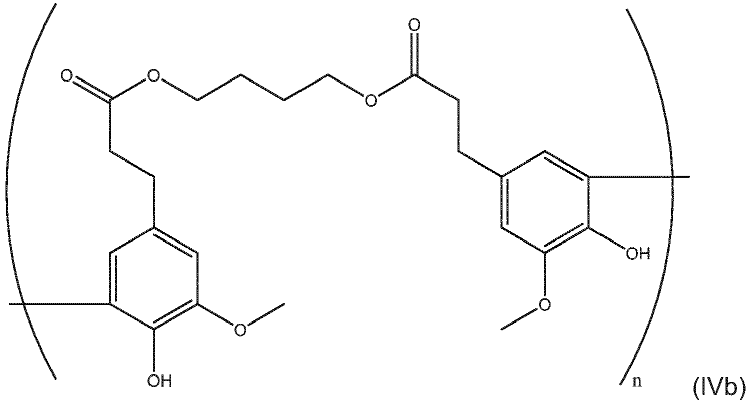 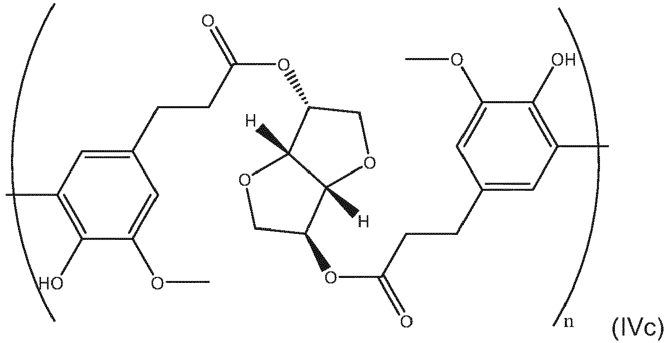 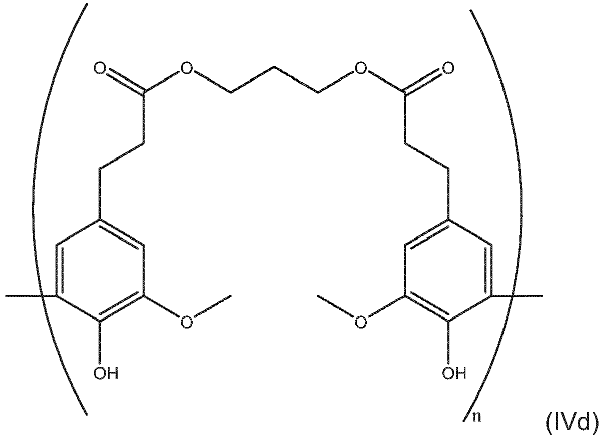 où n représente un nombre entier compris entre 2 et 100.

9. Polymère selon l'une quelconque des revendications 1 à 7, répondant à la formule générale (VII) : 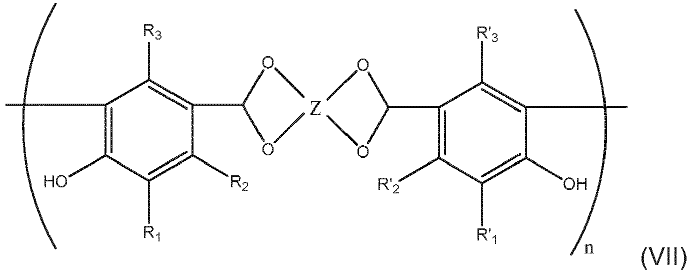 dans laquelle n représente un nombre entier compris entre 2 et 100.

10. Procédé de synthèse d'un polymère phénolique selon l'une quelconque des revendications 1 à 9, **caractérisé en ce qu'**il comprend une étape d'oligomérisation d'un ou de plusieurs macropolyphénol(s) répondant chacun à la formule générale (I) catalysée par une enzyme de type oxydase.

11. Procédé selon la revendication 10, selon lequel ladite enzyme est une laccase.

12. Procédé selon l'une quelconque des revendications 10 à 11, selon lequel ladite étape d'oligomérisation est réalisée en solution aqueuse, dans au moins une des conditions suivantes :
- à température comprise entre 0 et 75 °C,
- à pH compris entre 3 et 8,
- ladite solution aqueuse comportant de 0 à 80 % (v/v) d'un solvant organique.

13. Procédé selon l'une quelconque des revendications 10 à 11, selon lequel ladite étape d'oligomérisation est réalisée dans un liquide ionique.

14. Composition comprenant un polymère phénolique selon l'une quelconque des revendications 1 à 9.

15. Pièce dont une surface est revêtue d'une couche formée à base d'un polymère selon l'une quelconque des revendications 1 à 9.

16. Utilisation d'un polymère selon l'une quelconque des revendications 1 à 9, en tant qu'agent antioxydant, agent chélatant, agent antimicrobien et/ou agent plastifiant.

## Patentansprüche

1. Phenolpolymer, das durch katalysierte Oligomerisierung durch ein Enzym vom Typ Oxydase erhalten werden kann, eines oder mehrerer Macropolyphenol(e), die jeweils der allgemeinen Formel (I) entsprechen: 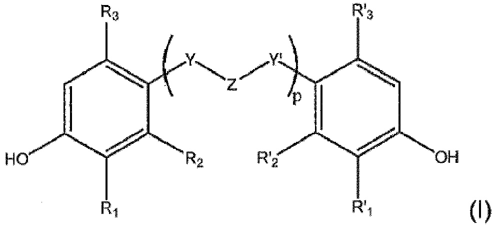 wobei:
p eine ganze Zahl im Bereich zwischen 1 und 30 darstellt, R₁, R'₁, R₂, R'₂, R₃ und R'₃, identisch oder verschieden, jeweils ein Wasserstoffatom, ein Chloratom, ein Bromatom, ein Iodatom, ein Fluoratom, eine Gruppe Alkyl, Benzyl, Xalkyl, gegebenenfalls substituiert, Xbenzyl, gegebenenfalls substituiert, Xacyl, B(OR')₂, NHR', NO₂, SR'O oder SO₂R' darstellen,
wobei X N, O, S oder P darstellt
und R' eine Alkylgruppe oder eine Arylgruppe darstellt,
wobei R₁ und R'₁ kein Wasserstoffatom darstellen,
Y und Y', identisch oder verschieden, jeweils Folgendes darstellen:
entweder ein Sauerstoffatom, ein Schwefelatom oder eine entkonjugierende Gruppe, die weder Epoxydkern noch Aziridinkern noch Phenolgruppierung, die nicht auf allen ihren Kohlenstoffatomen substituiert ist, umfasst,
oder eine Gruppe, die der Formel (II) entspricht: 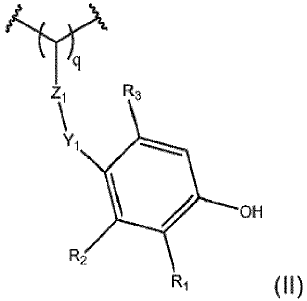
wobei:
q eine ganze Zahl im Bereich zwischen 1 und 8 darstellt,
Y₁ ein Sauerstoffatom, ein Schwefelatom oder eine entkonjugierende Gruppe darstellt, die weder Epoxydkern noch Aziridinkern noch Phenolgruppierung umfasst, die nicht auf allen ihren Kohlenstoffatomen substituiert ist,
und Z₁ ein Heteroatom oder eine Beabstandungsgruppe darstellt, die weder Epoxydkern noch Aziridinkern noch Phenolgruppierung, die nicht auf allen ihren Kohlenstoffatomen substituiert ist, noch Alkenylgruppe noch Alkynylgruppe umfasst,
und Z Folgendes darstellt:
entweder ein Heteroatom oder eine Beabstandungsgruppe, die weder Epoxydkern noch Aziridinkern noch Phenolgruppierung, die nicht auf allen ihren Kohlenstoffatomen substituiert ist, noch Alkenylgruppe noch Alkynylgruppe umfasst,
oder eine Gruppe, die der Formel (III) entspricht: 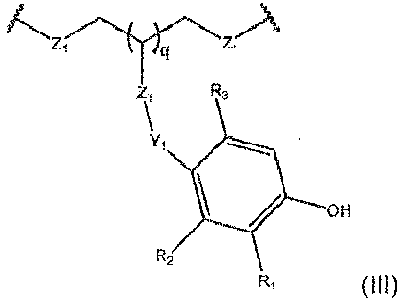 wobei q eine ganze Zahl zwischen 1 und 8 darstellt, wobei die Verbindungen zwischen den Macropolyphenolfragmenten mit der allgemeinen Formel (I) im Inneren des Polymers ausschließlich 5-5-Biarylbindungen sind.

2. Polymer nach Anspruch 1, wobei R₁ und/oder R'₁ ein lineares oder verzweigtes gesättigtes kohlenwasserstoffhaltiges Radikal darstellt/darstellen, umfassend von 1 bis 5 Kohlenstoffatome, oder eine Gruppe OR₄, wobei R₄ ein lineares oder verzweigtes gesättigtes kohlenwasserstoffhaltiges Radikal darstellt, umfassend von 1 bis 5 Kohlenstoffatome.

3. Polymer nach einem der Ansprüche 1 bis 2, wobei R₂, R₃, R'₂ und/oder R'₃ ein Wasserstoffatom darstellt/darstellen.

4. Polymer nach einem der Ansprüche 1 bis 3, wobei Y und/oder Y' eine Gruppe mit der allgemeinen Formel (V) darstellt/darstellen:
(CH₂)ₘ-X'- (V)
wobei:
m im Bereich zwischen 1 und 5 liegt,
und X' ein Sauerstoffatom oder ein Schwefelatom oder eine Gruppe, ausgewählt aus den Gruppen darstellt: 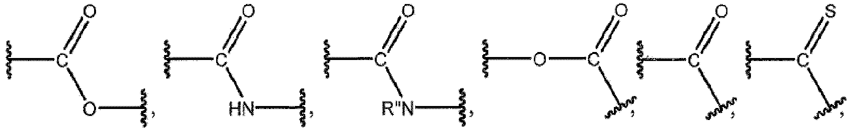 NR", NH oder SO₂, wobei R" eine Alkylgruppe oder eine Arylgruppe darstellt.

5. Polymer nach einem der Ansprüche 1 bis 3, wobei Y und Y' und gegebenenfalls Y₁ jeweils eine Gruppe der allgemeinen Formel (VI) darstellen:

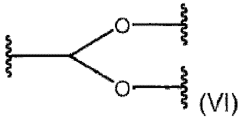

6. Polymer nach einem der Ansprüche 1 bis 5, wobei Z eine lineare oder verzweigte gesättigte kohlenwasserstoffhaltige Gruppe, gegebenenfalls substituiert, darstellt, umfassend von 1 bis 6 Kohlenstoffatome, die ein oder mehrere Heteroatome umfassen kann, oder eine gesättigte zyklische kohlenwasserstoffhaltige Gruppe, gegebenenfalls substituiert, umfassend von 1 bis 6 Kohlenstoffatome, die einen einzigen Zyklus oder mehrere kondensierte Zyklen umfassen kann, und ein oder mehrere Heteroatome umfassen kann.

7. Polymer nach einem der Ansprüche 1 bis 6, das der allgemeinen Formel (IV) entspricht: 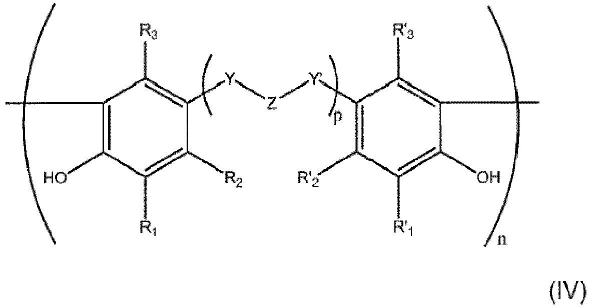 wobei n eine ganze Zahl darstellt, die im Bereich zwischen 2 und 100 liegt.

8. Polymer nach einem der Ansprüche 1 bis 7, das einer der Formeln (IVa), (IVb), (IVc) oder (IVd) entspricht: 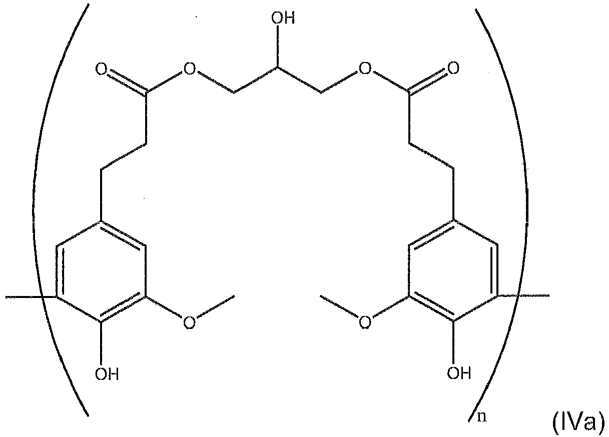 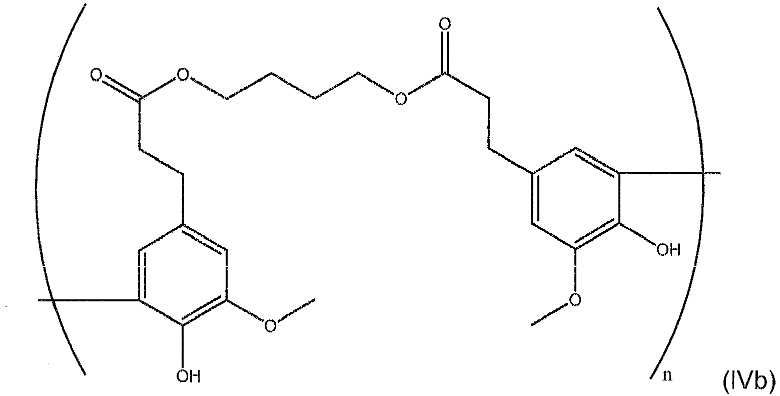 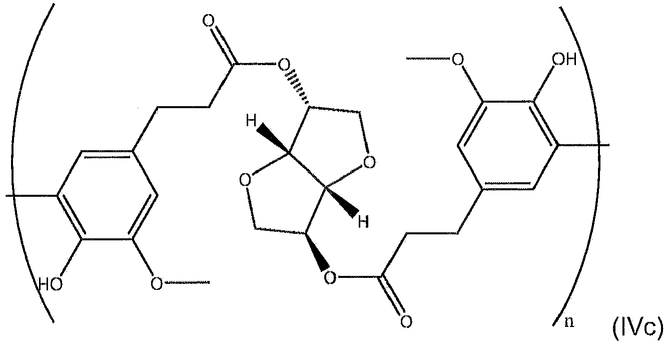 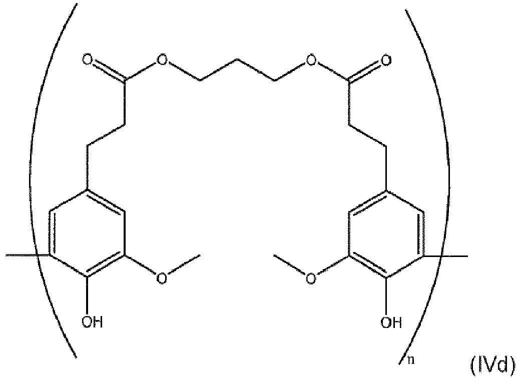 wobei n eine ganze Zahl darstellt, die im Bereich zwischen 2 und 100 liegt.

9. Polymer nach einem der Ansprüche 1 bis 7, das der allgemeinen Formel (VII) entspricht: 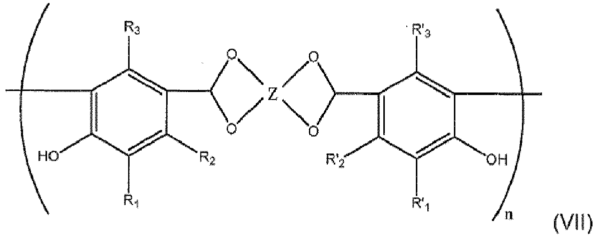 wobei n eine ganze Zahl darstellt, die im Bereich zwischen 2 und 100 liegt.

10. Verfahren zur Synthese eines Phenolpolymers nach einem der Ansprüche 1 bis 9, **dadurch gekennzeichnet, dass** es einen Schritt des Oligomerisierens eines oder mehrerer Macropolyphenole umfasst, die jeweils der allgemeinen Formel (I) entsprechen, katalysiert durch ein Enzym vom Typ Oxydase.

11. Verfahren nach Anspruch 10, nach dem das Enzym eine Laccase ist.

12. Verfahren nach einem der Ansprüche 10 bis 11, nach dem der Schritt des Oligomerisierens in wässriger Lösung unter mindestens einer der folgenden Bedingungen durchgeführt wird:
- bei einer Temperatur im Bereich zwischen 0 und 75 °C,
- bei einem pH-Wert im Bereich zwischen 3 und 8,
- wobei die wässrige Lösung von 0 bis 80 % (v/v) eines organischen Lösemittels umfasst.

13. Verfahren nach einem der Ansprüche 10 bis 11, nach dem der Schritt des Oligomerisierens in einer ionischen Flüssigkeit durchgeführt wird.

14. Zusammensetzung, umfassend ein Phenolpolymer nach einem der Ansprüche 1 bis 9.

15. Werkstück, von dem eine Oberfläche mit einer Schicht bedeckt ist, die auf der Grundlage eines Polymers nach einem der Ansprüche 1 bis 9 gebildet ist.

16. Verwendung eines Polymers nach einem der Ansprüche 1 bis 9 als Antioxidationsmittel, Chelatbildner, antimikrobielles Mittel und/oder Plastifizierungsmittel.

## Claims

1. A phenol polymer obtainable by oligomerization catalyzed by an enzyme of oxidase type, of one or more macropolyphenol(s) each corresponding to general formula (I): 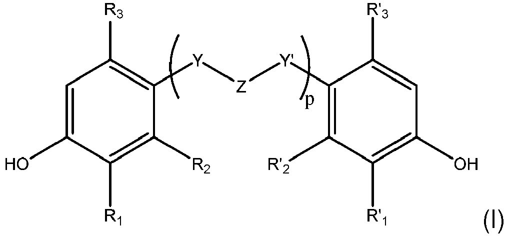 wherein:
p represents an integer between 1 and 30,
R₁, R'₁, R₂, R'₂, R₃ and R'₃, which may be identical or different, each represent a hydrogen atom, a chlorine atom, a bromine atom, an iodine atom, a fluorine atom, or an alkyl, benzyl, Xalkyl, optionnally substituted, Xbenzyl, optionnally substituted, Xacyl, B(OR')₂, NHR', NO₂, SR'O or SO₂R' group,
where X represents N, O, S or P
and R' represents an alkyl group or an aryl group,
R₁ and R'₁ do not representing a hydrogen atom,
Y and Y', which may be identical or different, each represent:
either an oxygen atom, a sulfur atom or a deconjugating group comprising neither an epoxide ring, nor an aziridine ring, nor a phenol group which is not substituted on all its carbon atoms,
or a group corresponding to formula (II): 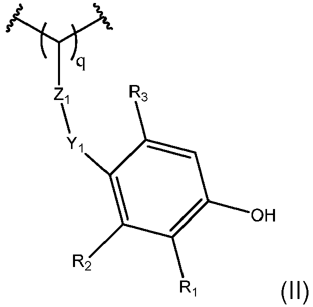
wherein:
q represents an integer between 1 and 8,
Y₁ represents an oxygen atom, a sulfur atom or a deconjugating group comprising neither an epoxide ring, nor an aziridine ring,
nor a phenol group which is not substituted on all its carbon atoms,
and Z₁ represents a heteroatom or a spacer group comprising neither an epoxide ring, nor an aziridine ring, nor a phenol group which is not substituted on all its carbon atoms, nor an alkenyl group, nor an alkynyl group,
and Z represents:
either a heteroatom or a spacer group comprising neither an epoxide ring, nor an aziridine ring, nor a phenol group which is not substituted on all its carbon atoms, nor an alkenyl group, nor an alkynyl group,
or a group corresponding to formula (III): 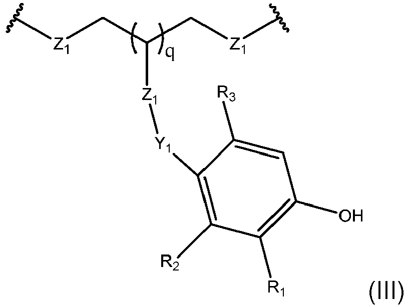 wherein q represents an integer between 1 and 8,
the bonds between the macropolyphenol fragments of general formula (I) within said polymer being exclusively 5,5-biaryl bonds.

2. The polymer as claimed in claim 1, wherein R₁ and/or R'₁ represent(s) a linear or branched, saturated hydrocarbon-based radical comprising from 1 to 5 carbon atoms, or an OR₄ group, where R₄ represents a linear or branched, saturated hydrocarbon-based radical comprising from 1 to 5 carbon atoms.

3. The polymer as claimed in either one of claims 1 and 2, wherein R₂, R₃, R'₂ and/or R'₃ represent(s) a hydrogen atom.

4. The polymer as claimed in any one of claims 1 to 3, wherein Y and/or Y' represent(s) a group of general formula (V):
(CH₂)ₘ-X'- (V)
wherein:
m is between 1 and 5,
and X' represents an oxygen atom or a sulfur atom or a group chosen from the groups: 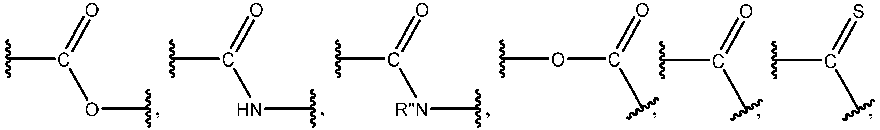
NR", NH or SO₂, where R" represents an alkyl group or an aryl group.

5. The polymer as claimed in any one of claims 1 to 3, wherein Y and Y', and optionnally Y₁, each represent a group of general formula (VI):

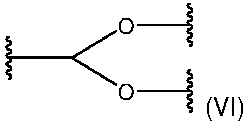

6. The polymer as claimed in any one of claims 1 to 5, wherein Z represents a linear or branched, saturated hydrocarbon-based group, optionnally substituted, comprising from 1 to 6 carbon atoms, which can comprise one or more heteroatoms, or a saturated cyclic hydrocarbon-based group, optionnally substituted, comprising from 1 to 6 carbon atoms, which can comprise one ring or several condensed rings, and which can comprise one or more heteroatoms.

7. The polymer as claimed in any one of claims 1 to 6, corresponding to general formula (IV): 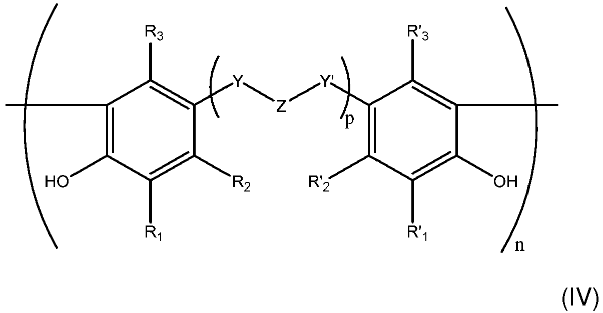 wherein n represents an integer between 2 and 100.

8. The polymer as claimed in any one of claims 1 to 7, corresponding to one of formulae (IVa), (IVb), (IVc) and (IVd): 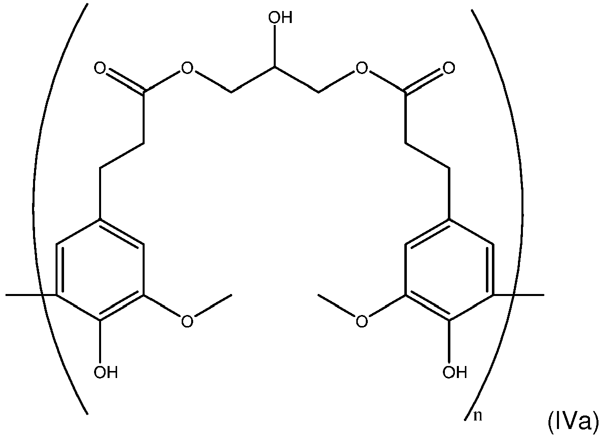 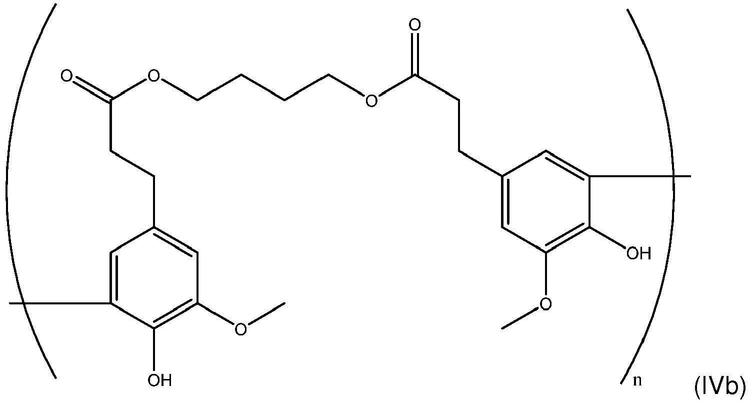 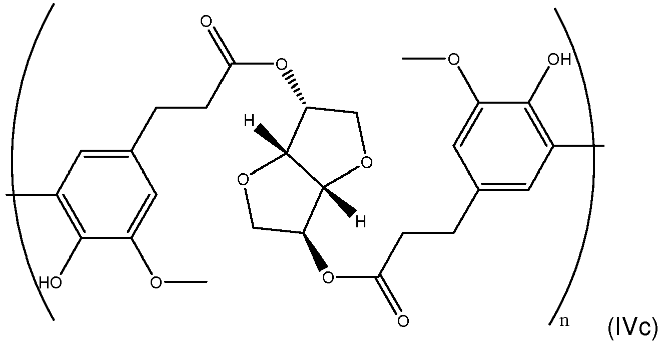 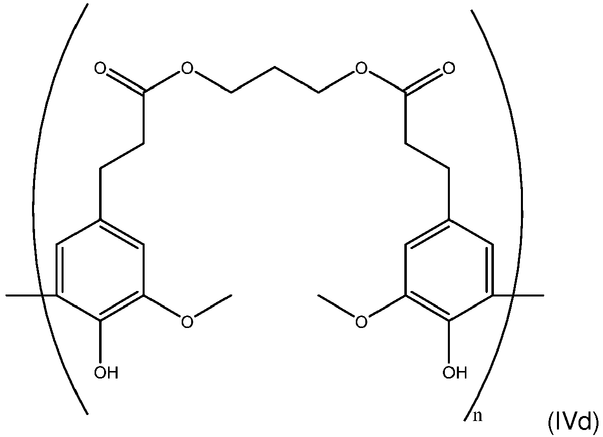 wherein n represents an integer between 2 and 100.

9. The polymer as claimed in any one of claims 1 to 7, corresponding to general formula (VII): 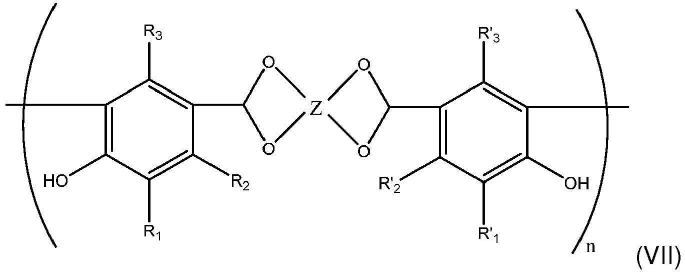 wherein n represents an integer between 2 and 100.

10. A process for synthesizing a phenol polymer as claimed in any one of claims 1 to 9, **characterized in that** it comprises a step of oligomerization of one or more macropolyphenol(s) each corresponding to general formula (I), catalyzed by an enzyme of oxidase type.

11. The process as claimed in claim 10, wherein said enzyme is a laccase.

12. The process as claimed in either one of claims 10 and 11, wherein said oligomerization step is carried out in an aqueous solution, under at least one of the following conditions:
- at a temperature of between 0 and 75°C,
- at a pH of between 3 and 8,
- said aqueous solution comprising from 0 to 80% (v/v) of an organic solvent.

13. The process as claimed in either one of claims 10 and 11, wherein said oligomerization step is carried out in an ionic liquid.

14. A composition comprising a phenol polymer as claimed in any one of claims 1 to 9.

15. A part, the surface of which is coated with a layer made up of a polymer as claimed in any one of claims 1 to 9.

16. The use of a polymer as claimed in any one of claims 1 to 9, as an antioxidant, chelating agent,antimicrobial agent and/or plasticizer.
